# NEUE EUROPÄISCHE PATENTSCHRIFT

(11) **EP 1 888 642 B2**
(45) Veröffentlichungstag und Bekanntmachung des Hinweises auf die Entscheidung über den Einspruch: **19.10.2016**
(45) Hinweis auf die Patenterteilung: 02.10.2013
(21) Anmeldenummer: 06742825.0
(22) Anmeldetag: 08.05.2006
(51) Int. Cl.: C07K 16/22, C07K 7/00

(54) **BINDUNGSPARTNER DES PLAZENTALEN WACHSTUMSFAKTORS INSBESONDERE GEGEN DEN PLAZENTALEN WACHSTUMSFAKTOR GERICHTETE ANTIKÖRPER, IHRE HERSTELLUNG UND VERWENDUNG**
BINDING PARTNERS OF THE PLACENTAL GROWTH FACTOR, ESPECIALLY ANTIBODIES DIRECTED AGAINST THE PLACENTAL GROWTH FACTOR, PRODUCTION AND USE THEREOF
PARTENAIRES DE LIAISON DU FACTEUR DE CROISSANCE PLACENTAIRE, NOTAMMENT ANTICORPS DIRIGES CONTRE LE FACTEUR DE CROISSANCE PLACENTAIRE, LEUR PRODUCTION ET LEUR UTILISATION

(30) Priorität: 09.05.2005 DE 102005022047
(43) Veröffentlichungstag der Anmeldung: 20.02.2008
(60) Teilanmeldung: 11002653.1
(73) Patentinhaber: Siemens Healthcare Diagnostics Products GmbH, 35041 Marburg (DE)
(72) Erfinder: VITZTHUM, Frank, 35094 Lahntal-Sterzhausen (DE); TEIGELKAMP, Stefan, 35096 Niederwalgern (DE); ALTHAUS, Harald, 35083 Wetter (DE)
(86) Internationale Anmeldenummer: PCT/EP2006/004278
(87) Internationale Veröffentlichungsnummer: WO 2006/128553

(56) Entgegenhaltungen:
- WO-A-99/24056
- WO-A-2005/077007
- MAYNARD SHARON E ET AL: "Excess placental soluble fms-like tyrosine kinase 1 (sFlt1) may contribute to endothelial dysfunction, hypertension, and proteinuria in preeclampsia" JOURNAL OF CLINICAL INVESTIGATION, NEW YORK, NY, US, Bd. 111, Nr. 5, März 2003 (2003-03), Seiten 649-658, XP002314744 ISSN: 0021-9738 in der Anmeldung erwähnt
- MAGLIONE D ET AL: "Isolation of a human placenta cDNA coding for a protein related to the vascular permeability factor" PROCEEDINGS OF THE NATIONAL ACADEMY OF SCIENCES OF USA, NATIONAL ACADEMY OF SCIENCE, WASHINGTON, DC, US, Bd. 88, Oktober 1991 (1991-10), Seiten 9267-9271, XP002080555 ISSN: 0027-8424
- SHALINI IYER AND RAVI ACHARYA: TCM, vol. 12, no. 3, 1 January 2002 (2002-01-01), pages 128-134,

### SEQUENCE LISTING

<110> Dade Behring Marburg GmbH
<120> Bindungspartner des Plazentalen wachstumsfaktors, insbesondere gegen Plazentalen wachstumfaktor gerichtete Antikörper, ihre Herstellung und Verwendung
<130> 05/016 DBM
<150> DE 10 2005 022 047.9
   <151> 2005-05-09
<160> 63
<170> PatentIn version 3.3
<210> 1
   <211> 149
   <212> PRT
   <213> Artificial sequence
<220>
   <223> Peptide
<400> 1
   Met Pro Val Met Arg Leu Phe Pro Cys Phe Leu Gln Leu Leu Ala Gly
   1               5                   10                  15
   Leu Ala Leu Pro Ala Val Pro Pro Gln Gln Trp Ala Leu Ser Ala Gly
               20                  25                  30
   Asn Gly Ser Ser Glu Val Glu Val Val Pro Phe Gln Glu Val Trp Gly
           35                  40                  45
   Arg Ser Tyr Cys Arg Ala Leu Glu Arg Leu Val Asp Val Val Ser Glu
       50                  55                  60
   Tyr Pro Ser Glu Val Glu His Met Phe Ser Pro Ser Cys Val Ser Leu
   65                  70                  75                  80
   Leu Arg Cys Thr Gly Cys Cys Gly Asp Glu Asn Leu His Cys Val Pro
                   85                  90                  95
   Val Glu Thr Ala Asn Val Thr Met Gln Leu Leu Lys Ile Arg Ser Gly
               100                 105                 110
   Asp Arg Pro Ser Tyr Val Glu Leu Thr Phe Ser Gln His Val Arg Cys
           115                 120                 125
   Glu Cys Arg Pro Leu Arg Glu Lys Met Lys Pro Glu Arg Cys Gly Asp
       130                 135                 140
   Ala Val Pro Arg Arg
   145
<210> 2
   <211> 132
   <212> PRT
   <213> Artificial Sequence
<220>
   <223> Peptide
<400> 2
   Ala Leu Pro Ala Val Pro Pro Gln Gln Trp Ala Leu Ser Ala Gly Asn
   1 5 10 15
   Gly Ser Ser Glu Val Glu Val Val Pro Phe Gln Glu Val Trp Gly Arg
   20 25 30
   Ser Tyr Cys Arg Ala Leu Glu Arg Leu Val Asp Val Val Ser Glu Tyr
   35 40 45
   Pro Ser Glu Val Glu His Met Phe Ser Pro Ser Cys Val Ser Leu Leu
   50 55 60
   Arg Cys Thr Gly Cys Cys Gly Asp Glu Asn Leu His Cys Val Pro Val
   65 70 75 80
   Glu Thr Ala Asn Val Thr Met Gln Leu Leu Lys Ile Arg Ser Gly Asp
   85 90 95
   Arg Pro Ser Tyr Val Glu Leu Thr Phe Ser Gln His Val Arg Cys Glu
   100 105 110
   Cys Arg Pro Leu Arg Glu Lys Met Lys Pro Glu Arg Cys Gly Asp Ala
   115 120 125
   Val Pro Arg Arg
   130
<210> 3
   <211> 132
   <212> PRT
   <213> Artificial Sequence
<220>
   <223> Peptide
<400> 3
   Met Leu Pro Ala Val Pro Pro Gln Gln Trp Ala Leu Ser Ala Gly Asn
   1 5 10 15
   Gly Ser Ser Glu Val Glu Val Val Pro Phe Gln Glu Val Trp Gly Arg
   20 25 30
   Ser Tyr Cys Arg Ala Leu Glu Arg Leu Val Asp Val Val Ser Glu Tyr
   35 40 45
   Pro Ser Glu Val Glu His Met Phe Ser Pro Ser Cys Val Ser Leu Leu
   50 55 60
   Arg Cys Thr Gly Cys Cys Gly Asp Glu Asn Leu His Cys Val Pro Val
   65 70 75 80
   Glu Thr Ala Asn Val Thr Met Gln Leu Leu Lys Ile Arg Ser Gly Asp
   85 90 95
   Arg Pro Ser Tyr Val Glu Leu Thr Phe Ser Gln His Val Arg Cys Glu
   100 105 110
   Cys Arg Pro Leu Arg Glu Lys Met Lys Pro Glu Arg Cys Gly Asp Ala
   115 120 125
   Val Pro Arg Arg
   130
<210> 4
   <211> 153
   <212> PRT
   <213> Artificial Sequence
<220>
   <223> Peptide
<400> 4
   Ala Leu Pro Ala Val Pro Pro Gln Gln Trp Ala Leu Ser Ala Gly Asn
   1 5 10 15
   Gly Ser Ser Glu Val Glu Val Val Pro Phe Gln Glu Val Trp Gly Arg
   20 25 30
   Ser Tyr Cys Arg Ala Leu Glu Arg Leu Val Asp Val Val Ser Glu Tyr
   35 40 45
   Pro Ser Glu Val Glu His Met Phe Ser Pro Ser Cys Val Ser Leu Leu
   50 55 60
   Arg Cys Thr Gly Cys Cys Gly Asp Glu Asn Leu His Cys Val Pro Val
   65 70 75 80
   Glu Thr Ala Asn Val Thr Met Gln Leu Leu Lys Ile Arg Ser Gly Asp
   85 90 95
   Arg Pro Ser Tyr Val Glu Leu Thr Phe Ser Gln His Val Arg Cys Glu
   100 105 110
   Cys Arg Pro Leu Arg Glu Lys Met Lys Pro Glu Arg Arg Arg Pro Lys
   115 120 125
   Gly Arg Gly Lys Arg Arg Arg Glu Lys Gln Arg Pro Thr Asp Cys His
   130 135 140
   Leu Cys Gly Asp Ala Val Pro Arg Arg
   145 150
<210> 5
   <211> 204
   <212> PRT
   <213> Artificial Sequence
<220>
   <223> Peptide
<400> 5
   Ala Leu Pro Ala Val Pro Pro Gln Gln Trp Ala Leu Ser Ala Gly Asn
   1 5 10 15
   Gly Ser Ser Glu Val Glu Val Val Pro Phe Gln Glu Val Trp Gly Arg
   20 25 30
   Ser Tyr Cys Arg Ala Leu Glu Arg Leu Val Asp Val Val Ser Glu Tyr
   35 40 45
   Pro Ser Glu Val Glu His Met Phe Ser Pro Ser Cys Val Ser Leu Leu
   50 55 60
   Arg Cys Thr Gly Cys Cys Gly Asp Glu Asn Leu His Cys Val Pro Val
   65 70 75 80
   Glu Thr Ala Asn Val Thr Met Gln Leu Leu Lys Ile Arg Ser Gly Asp
   85 90 95
   Arg Pro Ser Tyr Val Glu Leu Thr Phe Ser Gln His Val Arg Cys Glu
   100 105 110
   Cys Arg His Ser Pro Gly Arg Gln Ser Pro Asp Met Pro Gly Asp Phe
   115 120 125
   Arg Ala Asp Ala Pro Ser Phe Leu Pro Pro Arg Arg Ser Leu Pro Met
   130 135 140
   Leu Phe Arg Met Glu Trp Gly Cys Ala Leu Thr Gly Ser Gln Ser Ala
   145 150 155 160
   Val Trp Pro Ser Ser Pro Val Pro Glu Glu Ile Pro Arg Met His Pro
   165 170 175
   Gly Arg Asn Gly Lys Lys Gln Gln Arg Lys Pro Leu Arg Glu Lys Met
   180 185 190
   Lys Pro Glu Arg Cys Gly Asp Ala Val Pro Arg Arg
   195 200
<210> 6
   <211> 225
   <212> PRT
   <213> Artificial Sequence
<220>
   <223> Peptide
<400> 6
   Ala Leu Pro Ala Val Pro Pro Gln Gln Trp Ala Leu Ser Ala Gly Asn
   1 5 10 15
   Gly Ser Ser Glu Val Glu Val Val Pro Phe Gln Glu Val Trp Gly Arg
   20 25 30
   Ser Tyr Cys Arg Ala Leu Glu Arg Leu Val Asp Val Val Ser Glu Tyr
   35 40 45
   Pro Ser Glu Val Glu His Met Phe Ser Pro Ser Cys Val Ser Leu Leu
   50 55 60
   Arg Cys Thr Gly Cys Cys Gly Asp Glu Asn Leu His Cys Val Pro Val
   65 70 75 80
   Glu Thr Ala Asn Val Thr Met Gln Leu Leu Lys Ile Arg Ser Gly Asp
   85 90 95
   Arg Pro Ser Tyr Val Glu Leu Thr Phe Ser Gln His Val Arg Cys Glu
   100 105 110
   Cys Arg His Ser Pro Gly Arg Gln Ser Pro Asp Met Pro Gly Asp Phe
   115 120 125
   Arg Ala Asp Ala Pro Ser Phe Leu Pro Pro Arg Arg Ser Leu Pro Met
   130 135 140
   Leu Phe Arg Met Glu Trp Gly Cys Ala Leu Thr Gly Ser Gln Ser Ala
   145 150 155 160
   Val Trp Pro Ser Ser Pro Val Pro Glu Glu Ile Pro Arg Met His Pro
   165 170 175
   Gly Arg Asn Gly Lys Lys Gln Gln Arg Lys Pro Leu Arg Glu Lys Met
   180 185 190
   Lys Pro Glu Arg Arg Arg Pro Lys Gly Arg Gly Lys Arg Arg Arg Glu
   195 200 205
   Lys Gln Arg Pro Thr Asp Cys His Leu Cys Gly Asp Ala Val Pro Arg
   210 215 220
   Arg
   225
<210> 7
   <211> 30
   <212> PRT
   <213> Artificial sequence
<220>
   <223> Peptide
<400> 7
   Ser Ala Gly Asn Gly Ser Ser Glu Val Glu Val Val Pro Phe Gln Glu
   1 5 10 15
   Val Trp Gly Arg Ser Tyr Cys Arg Ala Leu Glu Arg Leu Val
   20 25 30
<210> 8
   <211> 9
   <212> PRT
   <213> Artificial Sequence
<220>
   <223> Peptide
<400> 8
   Phe Gln Glu Val Trp Gly Arg Ser Tyr
   1 5
<210> 9
   <211> 12
   <212> PRT
   <213> Artificial Sequence
<220>
   <223> Peptide
<400> 9
   Ser Ala Gly Asn Gly Ser Ser Glu Val Glu Val Val
   1 5 10
<210> 10
   <211> 13
   <212> PRT
   <213> Artificial Sequence
<220>
   <223> Peptide
<400> 10
   Ser Ala Gly Asn Gly Ser Ser Glu Val Glu Val Val Lys
   1 5 10
<210> 11
   <211> 12
   <212> PRT
   <213> Artificial Sequence
<220>
   <223> Peptide
<400> 11
   Val Val Pro Phe Gln Glu Val Trp Gly Arg Ser Tyr
   1 5 10
<210> 12
   <211> 13
   <212> PRT
   <213> Artificial Sequence
<220>
   <223> Peptide
<400> 12
   Val Val Pro Phe Gln Glu Val Trp Gly Arg Ser Tyr Lys
   1 5 10
<210> 13
   <211> 5
   <212> PRT
   <213> Artificial sequence
<220>
   <223> Peptide
<400> 13
   Glu Val Val Pro Phe
   1 5
<210> 14
   <211> 5
   <212> PRT
   <213> Artificial Sequence
<220>
   <223> Peptide
<400> 14
   Val Val Pro Phe Gln
   1 5
<210> 15
   <211> 5
   <212> PRT
   <213> Artificial Sequence
<220>
   <223> Peptide
<400> 15
   Val Pro Phe Gln Glu
   1               5
<210> 16
   <211> 5
   <212> PRT
   <213> Artificial Sequence
<220>
   <223> Peptide
<400> 16
   Pro Phe Gln Glu Val
   1               5
<210> 17
   <211> 5
   <212> PRT
   <213> Artificial Sequence
<220>
   <223> Peptide
<400> 17
   Phe Gln Glu Val Trp
   1               5
<210> 18
   <211> 5
   <212> PRT
   <213> Artificial Sequence
<220>
   <223> Peptide
<400> 18
   Gln Glu Val Trp Gly
   1               5
<210> 19
   <211> 5
   <212> PRT
   <213> Artificial Sequence
<220>
   <223> Peptide
<400> 19
   Glu Val Trp Gly Arg
   1 5
<210> 20
   <211> 5
   <212> PRT
   <213> Artificial Sequence
<220>
   <223> Peptide
<400> 20
   Val Trp Gly Arg Ser
   1 5
<210> 21
   <211> 5
   <212> PRT
   <213> Artificial Sequence
<220>
   <223> Peptide
<400> 21
   Trp Gly Arg Ser Tyr
   1 5
<210> 22
   <211> 5
   <212> PRT
   <213> Artificial Sequence
<220>
   <223> Peptide
<400> 22
   Gly Arg Ser Tyr Cys
   1 5
<210> 23
   <211> 5
   <212> PRT
   <213> Artificial Sequence
<220>
   <223> Peptide
<400> 23
   Arg Ser Tyr Cys Arg
   1 5
<210> 24
   <211> 5
   <212> PRT
   <213> Artificial Sequence
<220>
   <223> Peptide
<400> 24
   Ser Tyr Cys Arg Ala
   1 5
<210> 25
   <211>5 5
   <212> PRT
   <213> Artificial Sequence
<220>
   <223> Peptide
<400> 25
   Tyr Cys Arg Ala Leu
   1 5
<210> 26
   <211> 19
   <212> PRT
   <213> Artificial Sequence
<220>
   <223> Peptide
<400> 26
   Leu Arg Cys Thr Gly Cys Cys Gly Asp Glu Asn Leu His Cys Val Pro
   1 5 10 15
   Val Glu Thr
<210> 27
   <211> 5
   <212> PRT
   <213> Artificial Sequence
<220>
   <223> Peptide
<400> 27
   Gly Asp Glu Asn Leu
   1 5
<210> 28
   <211> 9
   <212> PRT
   <213> Artificial Sequence
<220>
   <223> Peptide
<400> 28
   Gly Cys Cys Gly Asp Glu Asn Leu His
   1 5
<210> 29
   <211> 10
   <212> PRT
   <213> Artificial Sequence
<220>
   <223> Peptide
<400> 29
   Gly Cys Cys Gly Asp Glu Asn Leu His Lys
   1 5 10
<210> 30
   <211> 5
   <212> PRT
   <213> Artificial Sequence
<220>
   <223> Peptide
<400> 30
   Gly Cys Cys Gly Asp
   1 5
<210> 31
   <211> 5
   <212> PRT
   <213> Artificial Sequence
<220>
   <223> Peptide
<400> 31
   Cys Cys Gly Asp Glu
   1 5
<210> 32
   <211> 5
   <212> PRT
   <213> Artificial Sequence
<220>
   <223> Peptide
<400> 32
   Cys Gly Asp Glu Asn
   1 5
<210> 33
   <211> 29
   <212> PRT
   <213> Artificial Sequence
<220>
   <223> Peptide
<400> 33
   Val Glu Thr Ala Asn Val Thr Met Gln Leu Leu Lys Ile Arg Ser Gly
   1 5 10 15
   Asp Arg Pro Ser Tyr Val Glu Leu Thr Phe Ser Gln His
   20 25
<210> 34
   <211> 13
   <212> PRT
   <213> Artificial Sequence
<220>
   <223> Peptide
<400> 34
   Gln Leu Leu Lys Ile Arg Ser Gly Asp Arg Pro Ser Tyr
   1 5 10
<210> 35
   <211> 5
   <212> PRT
   <213> Artificial Sequence
<220>
   <223> Peptide
<400> 35
   Gln Leu Leu Lys Ile
   1 5
<210> 36
   <211> 5
   <212> PRT
   <213> Artificial Sequence
<220>
   <223> Peptide
<400> 36
   Arg Pro Ser Tyr Val
   1               5
<210> 37
   <211> 12
   <212> PRT
   <213> Artificial Sequence
<220>
   <223> Peptide
<400> 37
   Arg Ser Gly Asp Arg Pro Ser Tyr Val Glu Leu Thr
   1               5                   10
<210> 38
   <211> 12
   <212> PRT
   <213> Artificial Sequence
<220>
   <223> Peptide
<400> 38
   Ser Gly Asp Arg Pro Ser Tyr Val Glu Leu Thr Lys
   1               5                   10
<210> 39
   <211> 9
   <212> PRT
   <213> Artificial Sequence
<220>
   <223> Peptide
<400> 39
   Val Glu Thr Ala Asn Val Thr Met Gln
   1               5
<210> 40
   <211> 5
   <212> PRT
   <213> Artificial Sequence
<220>
   <223> Peptide
<400> 40
   Val Glu Thr Ala Asn
   1 5
<210> 41
   <211> 5
   <212> PRT
   <213> Artificial Sequence
<220>
   <223> Peptide
<400> 41
   Thr Ala Asn Val Thr
   1 5
<210> 42
   <211> 5
   <212> PRT
   <213> Artificial Sequence
<220>
   <223> Peptide
<400> 42
   Asn Val Thr Met Gln
   1 5
<210> 43
   <211> 12
   <212> PRT
   <213> Artificial Sequence
<220>
   <223> Peptide
<400> 43
   Val Pro Val Glu Thr Ala Asn Val Thr Met Gln Leu
   1 5 10
<210> 44
   <211> 13
   <212> PRT
   <213> Artificial Sequence
<220>
   <223> Peptide
<400> 44
   Val Pro Val Glu Thr Ala Asn Val Thr Met Gln Leu Lys
   1 5 10
<210> 45
   <211> 20
   <212> PRT
   <213> Artificial Sequence
<220>
   <223> Peptide
<400> 45
   Thr Phe Ser Gln His Val Arg Cys Glu Cys Arg Pro Leu Arg Glu Lys
   1               5                   10                  15
   Met Lys Pro Glu
               20
<210> 46
   <211> 4
   <212> PRT
   <213> Artificial Sequence
<220>
   <223> Peptide
<400> 46
   Glu Cys Arg Pro
   1
<210> 47
   <211> 5
   <212> PRT
   <213> Artificial Sequence
<220>
   <223> Peptide
<400> 47
   Pro Leu Arg Glu Lys
   1               5
<210> 48
   <211> 36
   <212> PRT
   <213> Artificial Sequence
<220>
   <223> Peptide
<400> 48
   Arg Glu Lys Met Lys Pro Glu Arg Arg Arg Pro Lys Gly Arg Gly Lys
   1               5                   10                  15
   Arg Arg Arg Glu Lys Gln Arg Pro Thr Asp Cys His Leu Cys Gly Asp
               20                  25                  30
   Ala Val Pro Arg
           35
<210> 49
   <211> 5
   <212> PRT
   <213> Artificial Sequence
<220>
   <223> Peptide
<400> 49
   Met Lys Pro Glu Arg
   1               5
<210> 50
   <211> 5
   <212> PRT
   <213> Artificial Sequence
<220>
   <223> Peptide
<400> 50
   Lys Pro Glu Arg Arg
   1               5
<210> 51
   <211> 5
   <212> PRT
   <213> Artificial Sequence
<220>
   <223> Peptide
<400> 51
   Leu Cys Gly Asp Ala
   1               5
<210> 52
   <211> 86
   <212> PRT
   <213> Artificial Sequence
<220>
   <223> Peptide
<400> 52
   His Val Arg Cys Glu Cys Arg His Ser Pro Gly Arg Gln Ser Pro Asp
   1 5 10 15
   Met Pro Gly Asp Phe Arg Ala Asp Ala Pro Ser Phe Leu Pro Pro Arg
   20 25 30
   Arg Ser Leu Pro Met Leu Phe Arg Met Glu Trp Gly Cys Ala Leu Thr
   35 40 45
   Gly Ser Gln Ser Ala Val Trp Pro Ser Ser Pro Val Pro Glu Glu Ile
   50 55 60
   Pro Arg Met His Pro Gly Arg Asn Gly Lys Lys Gln Gln Arg Lys Pro
   65 70 75 80
   Leu Arg Glu Lys Met Lys
   85
<210> 53
   <211> 5
   <212> PRT
   <213> Artificial Sequence
<220>
   <223> Peptide
<400> 53
   Cys Glu Cys Arg His
   1 5
<210> 54
   <211> 5
   <212> PRT
   <213> Artificial Sequence
<220>
   <223> Peptide
<400> 54
   Glu Cys Arg His Ser
   1 5
<210> 55
   <211> 5
   <212> PRT
   <213> Artificial Sequence
<220>
   <223> Peptide
<400> 55
   Lys Pro Leu Arg Glu
   1 5
<210> 56
   <211> 25
   <212> PRT
   <213> Artificial Sequence
<220>
   <223> Peptide
<400> 56
   Asn Gly Lys Lys Gln Gln Arg Lys Pro Leu Arg Glu Lys Met Lys Pro
   1               5                   10                  15
   Glu Arg Arg Arg Pro Lys Gly Arg Gly
               20                  25
<210> 57
   <211> 5
   <212> PRT
   <213> Artificial Sequence
<220>
   <223> Peptide
<400> 57
   Gln Gln Arg Lys Pro
   1               5
<210> 58
   <211> 5
   <212> PRT
   <213> Artificial
<220>
   <223> oligopeptide
<400> 58
   Gln Arg Lys Pro Leu
   1               5
<210> 59
   <211> 5
   <212> PRT
   <213> Artificial Sequence
<220>
   <223> Peptide
<400> 59
   Arg Lys Pro Leu Arg
   1               5
<210> 60
   <211> 5
   <212> PRT
   <213> Artificial
<220>
   <223> oligopeptide
<400> 60
   Pro Glu Arg Arg Arg
   1               5
<210> 61
   <211> 5
   <212> PRT
   <213> Artificial
<220>
   <223> oligopeptide
<400> 61
   Glu Arg Arg Arg Pro
   1               5
<210> 62
   <211> 13
   <212> PRT
   <213> Artificial
<220>
   <223> oligopeptide
<400> 62
   Glu Lys Met Lys Pro Glu Arg Cys Gly Asp Ala Val Pro
   1               5                   10
<210> 63
   <211> 13
   <212> PRT
   <213> Artificial
<220>
   <223> oligopeptid
<400> 63
   Met Lys Pro Glu Arg Cys Gly Asp Ala Val Pro Arg Arg
   1               5                   10

## Beschreibung

Die Erfindung betrifft Bindungspartner des Plazentalen Wachstumsfaktors (Placental Growth Factor oder Placenta Growth Factor, P*l*GF) insbesondere gegen den Plazentalen Wachstumsfaktor gerichtete Antikörper, ihre Herstellung und Verwendung.

P*l*GF ist entscheidend an physiologischen und pathologischen Prozessen, insbesondere in der Angiogenese, beteiligt. Es spielt eine wichtige Rolle bei der Tumor-Progression, bei Nierenleiden, die insbesondere durch Diabetes Mellitus hervorgerufen werden, bei Psoriasis, entzündlichen Erkrankungen, insbesondere rheumatoide Arthritis, bei kardiovaskulären Erkrankungen u.a.m. [Iyer, S.; Leonidas, D. D.; Swaminathan, G. J.; Maglione, D.; Battisti, M.; Tucci, M.; Persico, M. G.; Acharya, K. R. J Biol Chem 2001, 276, (15), 12153-61. / Iyer, S.; Acharya, K. R. Trends Cardiovasc Med 2002, 12, (3), 128-34. / Heeschen, C.; Dimmeler, S.; Fichtlscherer, S.; Hamm, C. W.; Berger, J.; Simoons, M. L.; Zeiher, A. M. JAMA 2004, 291, (4), 435-41. / Yang, W.; Ahn, H.; Hinrichs, M.; Torry, R. J.; Torry, D. S. J Reprod Immunol 2003, 60, (1), 53-60.].

P*l*GF wird überwiegend in der Plazenta exprimiert und gehört zur "Cysteine-knot"-Proteinfamilie. P*l*GF liegt in verschiedenen Formen vor. Verschiedene Formen von P*l*GF sind (I) primäre Isoformen und (II) sekundäre Isoformen. (III) Zudem kann zwischen freiem P*l*GF (fP*l*GF) und gebundenem P*l*GF (gP*l*GF) unterschieden werden.

### (I) Primäre PlGF-Isoformen

Primäre PlGF-Isoformen werden durch die Primärsequenz, d.h. die Abfolge der Aminosäuren im Protein, bestimmt. Alternatives Splicing sowie posttranslationale Modifikationen, wie Glykosylierungen, Phosphorylierungen, Degradation (Abbauprodukte, Fragmente, etc.), Acetylierungen, etc., führen zu verschiedenen primären P*l*GF-Isoformen. Bisher wurden vier verschiedene primäre Isoformen des humanen P*l*GF, P*l*GF-1 (P*l*GF-131), P*l*GF-2 (P*l*GF152), P*l*GF-3 (P*l*GF-203) und P*l*GF-4 beschrieben.

Die Sequenz des P*l*GF-1-Vorläufers (Sequenznummer (SN) 1V) lautet wie folgt:
SN 1 V:
Sezerniertes P*l*GF-1 besitzt in der Regel nicht die Leader-Sequenz des P*l*GF-1-Vorläufers (P*l*GF-Precursor) und beginnt somit N-terminal mit Alanin (A) (in der Sequenz des P*l*GF-1-Vorläufers als A angegeben, siehe oben). Dies gilt in der Regel auch für die anderen primären P*l*GF-Isoformen.

Die Sequenz der primären P*l*GF-1-Isoform lautet somit wie folgt:
SN 1:

Diese Primärsequenz läßt mögliche Stellen für posttranslational Modifikationen erkennen und somit auch das Vorhandensein posttranslational modifizierter primärer Isoformen. Beispielsweise liegt im Allgemeinen in vivo die postranslational modifizierte primäre PlGF-Isoform des an Position 84 (Asparigin, N) glykosylierten P*l*GF-1 vor.

Als erste N-terminale Aminosäure der primären Isoform P*l*GF-1 wird statt Alanin häufig Methionin (M) angegeben. Dies bezieht sich im Allgemeinen auf rekombinantes, beispielsweise in *Escherichia coli* (*E. coli*) exprimiertes P*l*GF-1 (rP*l*GF-1), insbesondere auf das humane rP*l*GF-1 (rhP*l*GF-1). Hier wird AUG als Start-Codon verwendet, das Methionin codiert. Ein derartig, in *E. coli* exprimiertes, P*l*GF besitzt keine posttranslationalen Modifikationen, insbesondere auch keine Glykosylierungen.

Die Sequenz der rekombinanten, humanen, primären P*l*GF-1-Isoform wird im Allgemeinen folgendermaßen angegeben:
SN 1 RH:

Durch alternatives Splicing befindet sich in der P*l*GF-2-Isoform anstatt des Arginin (R) 124 die Sequenz *RRRPKGRGKRRREKQRPTDCHL*. Die Sequenz der primären PlGF-2-Isoform lautet daher:
SN 2:

Ein durch alternatives Splicing eingefügtes Insert von 72 Aminosäuren (HSPGRQSPDMPGDFRADAPSFLPPRRSLPMLFRMEWGCALTGSQSAVWPSSPVPEEI PRMHPGRNGKKQQRK) führt zur Sequenz der primären P*l*GF-3-Isoform:
SN 3:

Die primäre P*l*GF-4-Isoform beinhaltet sowohl Sequenzen der P*l*GF-2-Isoform (kursiv) als auch der P*l*GF-3-Isoform (unterstrichen):
SN 4:

### (II) Sekundäre PlGF-Isoformen

Sekundäre P*l*GF-Isoformen ergeben sich aus der Kombination primärer P*l*GF-Isoformen oder anderer Moleküle, insbesondere Moleküle, die homolog zu P*l*GF sind. Die primären P*l*GF-Isoformen bzw. weitere Moleküle stellen Untereinheiten der sekundären P*l*GF-Isoformen dar. Im Allgemeinen bestehen sekundäre P*l*GF-Isoformen aus zwei Untereinheiten. Damit liegt P*l*GF in der Regel als Dimer vor, d.h. als Homodimer oder Heterodimer. Homodimere bestehen aus zwei gleichen primären P*l*GF-Isoformen (Untereinheiten) wie P*l*GF-1 x P*l*GF-1, P*l*GF-2 x P*l*GF-2, P*l*GF-3 x P*l*GF-3 und P*l*GF-4 x P*l*GF-4. Heterodimere bestehen aus zwei unterschiedlichen primären P*l*GF-Isoformen oder einer primären P*l*GF-Isoform und einem anderen Molekül, insbesondere einem P*l*GF-Homologen wie dem Vascular Endothelial Growth Factor (VEGF) und dessen primären Isoformen. Mögliche Beispiele für Heterodimere sind P*l*GF-1 x P*l*GF-2, P*l*GF-3 x P*l*GF-4, P*l*GF-1 x VEGF, etc.

### (III) Freies PlGF (fPlGF) und gebundenes PlGF (gPlGF)

Da P*l*GF mit Bindungspartnern Komplexe bildet, sind neben den Isoformen auch die komplexierten bzw. gebundenen Formen von P*l*GF zu berücksichtigen. Prinzipiell sind die freien primären, insbesondere jedoch die freien sekundären P*l*GF-Isoformen (freies P*l*GF, fP*l*GF) von den komplexierten bzw. gebundenen Formen (gebundenes P*l*GF, gP*l*GF) zu unterscheiden. gP*l*GF ist beispielsweise Homodimeres P*l*GF-1, das komplexiert vorliegt. Hierbei kann es sich um einfache Komplexe handeln, d.h. ein P*l*GF-1-Homodimer ist an einem Rezeptor, beispielsweise dem membrangebundenen fms-ähnlichen Tyrosinkinase-Rezeptor-1 (mFlt-1) gebunden. Weitere Beispiele sind Komplexe mit dem löslichen Flt-1 (sFlt-1), mit Neurophilinen (NP; insbesondere NP-1 und NP-2), mit dem kinase domain-containing receptor/fetal liver kinase receptor (KDR/Flk-1, VEGFR-2), mit Heparinsulfatproteoglykanen (HSPG) sowie deren Isoformen, Homologen, Fragmenten und Abbauprodukten.Vielschichtig zusammengesetzte Komplexe aus mehreren und ggf. verschiedenen P*l*GF-Isoformen und mehreren und ggf. verschiedenen Bindungspartnern, insbesondere Rezeptoren, sind ebenfalls denkbar.

Die Funktion von P*l*GF wird durch die Bindung an den membrangebundenen bzw. löslichen fms-ähnlichen Tyrosinkinase-Rezeptor-1 (fms-like tyrosine kinase receptor-1 (Flt-1) oder Vascular Endothelial Growth Factor (VEGF)-Receptor-1 (VEGFR-1)) sowie den "kinase domain-containing receptor/fetal liver kinase receptor (KDR/Flk-1 bzw. VEGFR-2) vermittelt, moduliert bzw. inhibiert. Neben anderen möglichen Funktionen von P*l*GF ist insbesondere die Bindung von P*l*GF an membrangebundenem Flt-1 (mFlt-1) relevant. Diese führt zur mFlt-1-Transphosphorylierung und aktiviert somit Signaltransduktionskaskaden [Iyer, S.; Acharya, K. R. Trends Cardiovasc Med 2002, 12, (3), 128-34.].

Im Gegensatz hierzu wird davon ausgegangen, dass die Bindung von P*l*GF an sFlt-1 dazu dient, die physiologische Aktivität von P*l*GF zu reduzieren [Iyer, S.; Acharya, K. R. Trends Cardiovasc Med 2002, 12, (3), 128-34.]. Des Weiteren wird davon ausgegangen, dass die P*l*GF -Isoform eine Rolle spielt. P*l*GF -2, das unter Umständen mit der Membran assoziiert ist, besitzt ein kationisches Insert von 21 Aminosäuren am carboxyterminalen Ende. Durch die Bindung von anionischen, insbesondere polyanionischen, Substanzen wie Heparin, Heparinsulfatproteoglykanen, etc., können weitere Funktionen vermittelt werden. Entsprechende Auswirkungen kann auch die N-Glycosylierung von Asparigin (Asn) 84 haben sowie die Aminosäuresequenz, die sich im P*l*GF-3 befindet. Außerdem wird davon ausgegangen, dass die Bindung von P*l*GF and VEGF wiederum eine andere Auswirkung hat, da hier die VEGF Expression und damit dessen Aktivität negativ reguliert wird [Iyer, S.; Acharya, K. R. Trends Cardiovasc Med 2002, 12, (3), 128-34.]. Zusammenfassend bedeutet dies, dass die verschiedenen Formen von P*l*GF unterschiedliche Funktionen haben bzw. sich unterschiedlich auswirken.

### Stand der Technik

Für die derzeitigen Nachweisverfahren sowie Bindungspartner, insbesondere Antikörper, die momentan für analytische und diagnostische Zwecke eingesetzt werden, besteht das Problem, dass die verschiedenen Formen von P*l*GF nicht oder nicht effizient genug (nicht ausreichend spezifisch) unterschieden werden. Beispielsweise erkennt der "Anti-human P*l*GF Antibody" von R&D Systems, Inc. nicht ausschließlich bestimmte P*l*GF-Formen, insbesondere rhP*l*GF-1-Homodimer sondern auch das Heterodimer von rhP*l*GF und VEGF sowie rhP*l*GF-2 (R&D Systems Catalog Number: AF-264-PB bzw. DPG00 Produktbeschreibungen).

Des Weiteren wird nicht ausschließlich fP*l*GF oder gP*l*GF nachgewiesen, d.h. mit den bestehenden Antikörpern, wird nicht oder nicht effizient genug zwischen fP*l*GF und gP*l*GF unterschieden. Insbesondere ist der spezifische Nachweis von fP*l*GF unzureichend. Dies zeigt sich daran, dass rhFlt-1 in Form von rhFlt-1/Fc einen Einfluss auf die Bestimmung von P*l*GF hat (R&D Systems Catalog Number: DPG00). In der Literatur wird diese Unspezifität bestätigt [Maynard, S. E.; Min, J. Y.; Merchan, J.; Lim, K. H.; Li, J.; Mondal, S.; Libermann, T. A.; Morgan, J. P.; Sellke, F. W.; Stillman, I. E.; Epstein, F. H.; Sukhatme, V. P.; Karumanchi, S. A. J Clin Invest 2003, 111, (5), 649-58.]. Maynard et al. zeigen, dass der entsprechende R&D Systems ELISA (R&D Systems Catalog Number: AF-264-PB bzw. DPG00) zwar eine gewisse Spezifität für fP*l*GF erkennen läßt, die durchgeführten Untersuchungen, zeigen jedoch, dass diese Spezifität gering ist. Bei der Bestimmung von 0,5 ng/mL rhP*l*GF-1 is in Gegenwart von 0,5 ng/mL sFlt-1 eine Signalreduktion von lediglich etwa 12 % zu verzeichnen. Selbst bei 10fachem Überschuss von sFlt-1 (5 ng/mL) tritt lediglich eine Signalreduktion um den Faktor 2 auf. Eine deutlichere Signalreduktion würde bei einer höheren Spezifität der verwendeten Antikörper gegenüber fP*l*GF auftreten.

### Aufgabe und deren erfindungsgemäße Lösung

Der vorliegenden Erfindung lag also die Aufgabe zugrunde, Verfahren bzw. Komponenten bereitzustellen, die den spezifischen Nachweis von bestimmten P*l*GF-Formen, insbesondere mit Hilfe von spezifischen Bindungspartnern in Form von Antikörpern, ermöglichen.

Die Lösung dieser Aufgabe besteht in der Bereitstellung der in den Ansprüchen beschriebenen erfindungsgemäßen Gegenstände und Verfahren.

Insbesondere wird die Aufgabe durch die Bereitstellung von Antikörpern gelöst, die spezifisch an die primären Isoformen des P*l*GF binden. Diese Antikörper bilden die Basis für den immunologischen Nachweis und die Quantifizierung der primären und sekundären Isoformen sowie der freien bzw. gebundenen P*l*GF-Formen. Dies gilt insbesondere für biologische Materialien, insbesondere Plasmaproben, für diagnostische Applikationen. Therapeutische Applikationen sind ebenfalls denkbar.

Überraschenderweise können die verschiedenen Formen von P*l*GF wie im Folgenden beschrieben nachgewiesen werden. Im Folgenden wird die Wahl von Bindungspartnern und die von Substanzen zur Herstellung von spezifischen Bindungspartnern zum spezifischen Nachweis des freien P*l*GFs näher erläutert:

Prinzipiell sind spezifische Bindungspartner, insbesondere Antikörper und Proteine der Familie der Rezeptor-Tyrosinkinasen, insbesondere Flt-1, Flt-2, Flt-3, Flt-4, vorzugsweise Flt-1, Homologe, Fragmente und Abbauprodukte, die im Bereich der Rezeptor-bindenden Domäne an den Polen der sekundären P*l*GF-Isoformen binden, besonders für den Nachweis des fP*l*GF, insbesondere des nicht m/sFlt-1-gebundenen P*l*GFs geeignet.

Insbesondere sind spezifische Bindungspartner, insbesondere Antikörper, geeignet, die unter Verwendung von freien primären und sekundären P*l*GF-Isoformen, insbesondere sekundären P*l*GF-Isoformen, vorzugsweise P*l*GF-Homodimeren, besonders bevorzugt P*l*GF-1-Homodimer, insbesondere rhP*l*GF-1-Homodimer, vorzugsweise N-glykosyliertes rhP*l*GF-1-Homodimer hergestellt werden, beispielsweise durch Immunisierungen, und die bei der Charakterisierung zeigen, dass bei der Bindung Wechselwirkungen im Bereich der Rezeptorbindungsstelle eingegangen werden bzw. notwendig sind.

Die "Kopf-Schwanz"-Orientierung der Monomere (primäre P*l*GF-Isoformen) in Dimeren (sekundäre P*l*GF-Isoformen) führen, dazu, dass die Rezeptor-bindende Domäne sich jeweils an den Polen der P*l*GF-Dimere befindet. Die Rezeptorbindung findet an der Monomer-Monomer-Grenzfläche und nicht ausschließlich an einem Monomer statt.

Insbesondere Peptide, die nur die Sequenzinformation eines Monomers besitzen und damit nicht die gesamte Rezeptor-bindende Domäne umfassen, die aus beiden Monomeren aufgebaut ist, sind überraschenderweise als Immunisierungsantigene zur Herstellung von fP*l*GF-spezifischen Antikörpern besonders geeignet.

Peptide, die Aminosäuren beinhalten, die für Rezeptorinteraktionen relevant sind oder Peptide, die Sequenzbereiche in deren Nähe abdecken, sind besonders als Immunisierungsantigene geeignet. Die Aminosäuren, die insbesondere aber nicht ausschließlich für Flt-1-Rezeptorinteraktionen relevant sind, sind im Folgenden innerhalb der P*l*GF-1-Sequenz unterstrichen dargestellt. Die P*l*GF-1-Sequenz wurde beispielhaft zur Darstellung gewählt. Diese Aminosäuren sind auch bei den anderen P*l*GF-Isoformen für Rezeptorinteraktionen relevant.
SN 1:

Eine Ausnahme bilden E-112 und P-115, insbesondere jedoch P-115, die bei P*l*GF-3 und P*l*GF-4 bei den entsprechenden Rezeptorinteraktionen wahrscheinlich eine untergeordnete bzw. keine Rolle spielen, da zwischen diesen Aminosäuren das oben (SN 3 und SN 4) beschriebene Insert von 72 Aminosäuren liegt.

Folgende Peptide sind als Immunisierungsantigene zur Herstellung von fP*l*GF-spezifischen Antikörpern besonders geeignet (Immunisierungsantigennummern (IAN) 1-4):
IAN 1: SAGNGSSEVE VVPFQEVWGR SYCRALERLV
IAN 2: LRCTGCCGDE NLHCVPVET
IAN 3: VETANVTMQL LKIRSGDRP SYVELTFSQH
IAN 4: TFSQHVRCEC RPLREKMKPE RCGDAVPRR.

Immunisierungsantigene können auch Sequenzen aus verschiedenen Peptiden mit den Immunisierungsantigennummern IAN 1-4 beinhalten. Beispielsweise kann folgendes Peptid eingesetzt werden:
IAN 2/3: VPVETANVTM QL.

Im Folgenden werden spezifische Ausführungsformen näher erläutert:

Ein Gegenstand dieser Erfindung sind die Peptide bestehend aus einer der Aminosäuresequenzen IAN 1 oder IAN 3 oder aus einem Teil dieser Peptide, worin der Teil mindestens eine der Amniosäuresequenzen VVPFQEVWGRSY
(IAN: 1-1-2) oder RSGDRPSYVELT (IAN: 3-3-1) enthält.

Es sind auch Peptide, die 5 aufeinanderfolgende Aminosäuren der angegebenen Sequenzen beinhalten, d.h. zum Beispiel EVVPF (IAN: 1-2), VVPFQ (IAN:1-3), VPFQE (IAN: 1-4), PFQEV (IAN: 1-5), FQEVW (IAN: 1-6), QEVWG (IAN: 1-7), EVWGR (IAN: 1-8), VWGRS (IAN: 1-9), WGRSY (IAN: 1-10), GRSYC (IAN: 1-11), RSYCR (IAN: 1-12), SYCRA (IAN: 1-13), YCRAL (IAN: 1-14) oder GCCGD (IAN: 2-2), CCGDE (IAN: 2-3), CGDEN (IAN: 2-4), etc. bis hin zu PLREK (IAN: 4-2), offenbart.

Immunisierungsantigene können ungebunden und/oder trägergebunden zur Immunisierung eingesetzt werden. Um die Kopplung an typische Träger, beispielsweise Proteine, wie Ovalbumin, Albumin oder Keyhole Limpet Härnocyanin zu erleichtern, werden vorzugsweise Peptide synthetisiert, die ein Lysin beinhalten. Insbesondere sind hierfür folgende Peptide geeignet: SAGNGSSEVEVVK (IAN: 1-1-1K), RSGDRPSYVELTK (IAN: 3-3-1K), VPVETANVTMQLK (IAN: 2/3K), VVPFQEVWGRSYK (IAN: 1-1-2K), GCCGDENLHK (IAN: 2-1-1K).

Als Immunisierungsantigene können ebenfalls "multiple antigenic peptide systems" verwendet werden [Tam, J.P. Proc Natl Acad Sci USA 1988, 85, 5409-5413]. Insbesondere können 8-mere von IAN 1-1-1, IAN 3-3-1 oder IAN 2/3 verwendet werden:
IAN 1-1-1\8-mer: (SAGNGSSEVEVV)₈K₄K₂K-ßA
IAN 3-3-1\8-mer: (RSGDRPSYVELT)₈K₄K₂K-ßA
IAN 2/3\8-mer: (VPVETANVTMQL)₈K₄K₂K-ßA

Im Folgenden wird die Wahl von Substanzen zur Herstellung spezifischer Bindungspartner für den spezifischen Nachweis von gP*l*GF näher erläutert:

Unter Verwendung von gP*l*GF werden spezifische Antikörper, ermittelt bzw. hergestellt, beispielsweise durch Immunisierungen. Diese spezifischen Bindungspartner zeichnen sich bei deren Charakterisierung dadurch aus, dass bei der Bindung sowohl Wechselwirkungen mit dem P*l*GF als auch dem gebundenen Bindungspartner erfolgen.

Beispielsweise kann zur Herstellung von Antikörpern bei der Immunisierung P*l*GF eingesetzt werden, dass mit einem Bindungspartner einen Komplex bildet. Insbesondere kann es sich hierbei um P*l*GF-1-Homodimere handeln, die mit sFlt-1 komplexiert sind. Entsprechende Komplexe bestehend aus den jeweiligen Homologen, Fragmenten, etc. können ebenfalls eingesetzt werden.

Im Folgenden wird die Wahl von Substanzen zur Herstellung spezifischer Bindungspartner für den spezifischen Nachweis von posttranslational modifiziertem, insbesondere glykosyliertem P*l*GF näher erläutert.

Hierbei wird P*l*GF oder entsprechende Peptide mit bzw. ohne posttranslationaler Modifikation zur Herstellung von spezifischen Bindungspartnern, insbesondere Antikörpern eingesetzt. Die spezifischen Bindungspartner sind dann geeignet spezifisch die Abwesenheit bzw. das Vorhandensein von posttranslationalen Modifikationen nachzuweisen.

Beispielsweise können N-glykosylierte Peptide die die Sequenz VETANVTMQ (IAN: 3-4) oder Teile davon beinhalten, beispielsweise VETAN (IAN: 3-5), TANVT (IAN: 3-6), NVTMQ (IAN: 3-7), zur Herstellung von spezifischen Bindungspartnern, insbesondere Antikörpern, eingesetzt werden, die spezifisch für den Nachweis von an Asparigin 84 (N 84) glykosyliertem P*l*GF verwendet werden können. Neben diesen N-glykosylierten Peptiden ist es ebenfalls denkbar glykosyliertes P*l*GF, bzw. entsprechende Fragmente zu verwenden.

Zudem können durch den Einsatz entsprechender homologer, nicht glykosylierter Peptide, P*l*GF und entsprechender Fragmente Bindungspartner hergestellt werden, die beim spezifischen Nachweis von nicht glykosyliertem P*l*GF eingesetzt werden können.

Im Folgenden wird die Wahl von Substanzen zur Herstellung spezifischer Bindungspartner für den spezifischen Nachweis von P*l*GF-2 näher erläutert:

Hierbei werden primäre oder sekundäre P*l*GF-2-Isoformen, Fragmente hiervon oder entsprechende Peptide zur Herstellung von spezifischen Bindungspartnern, insbesondere Antikörpern eingesetzt. Insbesondere sind Peptide und deren Fragmente als Immunisierungsantigen zur Herstellung von P*l*GF-2-spezifischen Antikörpern besonders geeignet, die folgende Sequenz oder Teile hiervon beinhalten:
IAN 5: REKMKPE**RR RPKGRGKRRR EKQRPTDCHL** CGDAVPR.

Insbesondere bevorzugt sind Peptide einzusetzten, die die unterstrichene Sequenz oder Teile hiervon beinhalten.

Insbesondere sind Peptide, die 5 aufeinanderfolgende Aminosäuren der oben angegebenen Sequenz (IAN 5) beinhalten, besonders geeignet, beispielsweise MKPER (IAN: 5-1), KPERR (IAN: 5-2), etc. bis hin zu LCGDA (IAN: 5-3).

Neben den spezifischen Bindungspartnern für P*l*GF-2, insbesondere Antikörper, die durch die Immunisierung oder andere Prozeduren mit den oben beschriebenen Proteinen und Peptiden generiert werden, ist es ebenfalls möglich spezifische Bindungspartner wie anionische Verbindungen, insbesondere polyanionische Verbindungen, vorzugsweise Heparinverbindungen, insbesondere Heparinsulfatproteoglykane zu verwenden. In einer weiteren Ausführungsform werden Proteine, die zur Familie der Semaphorinrezeptoren zählen, insbesondere Neuropiline, vorzugsweise Neuropilin-1 (NP-1) und Neuropilin 2 (NP-2) als spezifische PlGF-2-Bindungspartner eingesetzt.

Im Folgenden wird die Wahl von Substanzen zur Herstellung spezifischer Bindungspartner für den spezifischen Nachweis von P*l*GF-3 näher erläutert:

Hierbei werden primäre oder sekundäre P*l*GF-2-Isoformen, Fragmente hiervon oder entsprechende Peptide zur Herstellung von spezifischen Bindungspartnern, insbesondere Antikörpern eingesetzt. Insbesondere sind Peptide und deren Fragmente als Immunisierungsantigen zur Herstellung von P*l*GF-3-spezifischen Antikörpern besonders geeignet, die folgende Sequenz oder Teile hiervon beinhalten:
IAN 6:

Insbesondere bevorzugt sind Peptide einzusetzen, die die unterstrichene Sequenz oder Teile hiervon beinhalten.

Insbesondere sind Peptide, die 5 aufeinanderfolgende Aminosäuren der angegebenen Sequenz IAN 6 beinhalten, d.h. zum Beispiel CECRH (IAN: 6-1), ECRHS (IAN: 6-2) etc. bis hin zu KPLRE (IAN: 6-3), besonders geeignet.

Im Folgenden wird die Wahl von Bindungspartnern und die von Substanzen zur Herstellung spezifischer Bindungspartner für den spezifischen Nachweis von P*l*GF-4 näher erläutert:

Hierbei werden primäre oder sekundäre P*l*GF-4-Isoformen, Fragmente hiervon oder entsprechende Peptide zur Herstellung von spezifischen Bindungspartnern, insbesondere Antikörpern eingesetzt. Insbesondere sind Peptide und deren Fragmente als Immunisierungsantigen zur Herstellung von P*l*GF-4-spezifischen Antikörpern besonders geeignet, die folgende Sequenz oder Teile hiervon beinhalten:
IAN 7: NGKKQQRKPL REKMKPERRR PKGRG.

Insbesondere bevorzugt sind Peptide einzusetzten, die die unterstrichenen Aminosäuren beinhalten.

Insbesondere sind Peptide die folgende Sequenzen beinhalten, geeignet: QQRKP (IAN: 7-1), QRKPL (IAN: 7-2), RKPLR (IAN: 7-3), KPLRE (IAN: 6-3), MKPER (IAN: 5-1), KPERR (IAN: 5-2), PERRR (IAN: 7-4), ERRRP (IAN: 7-5).

Da P*l*GF-4 sowohl spezifische PlGF-2-Sequenzen als auch spezifische PlGF-3-Sequenzen enthält, kann der Nachweis von P*l*GF-4 mit Hilfe erfindungsgemäßer spezifischer P*l*GF-2-Bindungspartner und P*l*GF-3-Bindungspartner, insbesondere Antikörper, die durch erfindungsgemäße Antigene, insbesondere Peptide, hergestellt werden, durchgeführt werden.

Im Folgenden wird die Wahl von Bindungspartnern und die von Substanzen zur Herstellung spezifischer Bindungspartner für den spezifischen Nachweis von P*l*GF/VEGF Heterodimeren näher erläutert:

Da P*l*GF/VEGF-Heterodimere sowohl spezifische P*l*GF-Sequenzen (P*l*GF 1-4) als auch spezifische VEGF-Sequenzen (VEGF-Isoformen) enthält, kann der Nachweis von P*l*GF/VEGF Heterodimeren, mit Hilfe erfindungsgemäßer spezifischer Antikörper, die durch Antigene, insbesondere Peptide, hergestellt werden, und VEGF-Antikörpern, durchgeführt werden.

Insbesondere können auch spezifische Antikörper, verwendet werden, die unter Verwendung von VEGF/P*l*GF-Heterodimeren ermittelt bzw. hergestellt wurden, beispielsweise durch Immunisierungen, und die bei der Charakterisierung zeigen, dass bei der Bindung sowohl Wechselwirkungen mit dem VEGF-Monomer als auch mit dem PlGF-Monomer eingegangen werden.

Eine bevorzugtes Verfahren zur Herstellung der erfindungsgemäßen Peptide, die u. a. als Immunisierungsantigene verwendet werden, ist die Festphasensynthese, wobei eine mehrfache Kopienzahl eines Peptids an einem Lysinkern synthetisiert wird [s. a. Tam J. P. (1988) Proc. Natl. Acad. Sci. USA 85: 5409-5413]. Die Peptidsynthese wird vorzugsweise mit Hilfe von Automaten, wie sie z. B. von Applied Biosystems (USA) angeboten werden, nach einem Standardprotokoll durchgeführt. Derartige multimere Peptide können weiterhin an ein Trägerprotein gebunden werden.

Unter einem "spezifischen Bindungspartner" ist ein Mitglied eines spezifischen Bindungspaares zu verstehen. Bei den Mitgliedern eines spezifischen Bindungspaares handelt es sich um zwei Moleküle, die jeweils mindestens eine zu einer Struktur des anderen Moleküls komplementäre Struktur aufweisen, wobei die beiden Moleküle sich über eine Bindung der komplementären Strukturen zu binden vermögen. Der Begriff Molekül umfasst auch Molekülkomplexe wie z.B. Enzyme, die aus Apo- und Coenzym bestehen, Proteine, die aus mehreren Untereinheiten bestehen, Lipoproteine bestehend aus Protein und Lipiden, etc. Spezifische Bindungspartner können natürlich vorkommende aber auch z.B. mittels chemischer Synthese, mikrobiologischer Techniken und/oder gentechnologischer Verfahren hergestellte Substanzen sein. Die folgende Aufzählung dient zur Verdeutlichung des Begriffs spezifischer Bindungspartner, ohne diesen jedoch auf diese Substanzen einzuschränken: thyroxinbindendes Globulin, steroidbindende Proteine, Antikörper, Antikörperfragmente, Designed Repeat Proteins, Protein Scaffolds, Ankyrine, Leucin-rich Repeats, Anticaline, Duocaline, Lipocaline, Affibodies®, Antigene, Haptene, Enzyme, Lektine, Nukleinsäuren, insbesondere Aptamere, Repressoren, Oligo- und Polynukleotide, Protein A, Protein G, Avidin, Streptavidin, Biotin, Komplementkomponente Clq, nukleinsäurebindende Proteine, etc. Spezifische Bindungspaare sind beispielsweise: Antikörper-Antigen, Antikörper-Hapten, Operator-Repressor, Nuclease-Nukleotid, Biotin-Avidin, Lektin-Polysaccharid, Steroidsteroidbindendes Protein, Wirkstoff-Wirkstoffrezeptor, Hormon-Hormonrezeptor, EnzymSubstrat, IgG-Protein A, komplementäre Oligo- oder Polynukleotide, etc.

Der Begriff "Peptide" im Sinne dieser Erfindung umfasst Säureamide, die bei Hydrolyse in Aminosäuren zerfallen, beispielsweise Aminosäurepolymere wie zum Beispiel Polypeptide, Oligopeptide, Proteine oder Proteinfragmente.

Die erfindungsgemäßen Peptide können als Immunisierungsantigen zur Herstellung der erfindungsgemäßen Antikörper oder auch zur affinitätschromatographischen Reinigung der erfindungsgemäßen Antikörper verwendet werden. Ferner können die erfindungsgemäßen Peptide auch in einem Verfahren zum quantitativen oder qualitativen Nachweis eines Analyten, bevorzugt der verschiedenen P*l*GF Formen, verwendet werden. Die erfindungsgemäßen Peptide können auch mit einer Festphase und/oder einer Komponente eines signalbildenden Systems, beispielsweise in einem Immuno-Assay, assoziiert sein.

Der Begriff "Antigene" umfasst monovalente und polyvalente Antigene. Ein polyvalentes Antigen ist ein Molekül oder ein Molekülkomplex, an das/den mehr als ein Immunoglobulin gleichzeitig binden kann, während bei einem monovalenten Antigen jeweils nur ein einziger Antikörper zur selben Zeit binden kann. Als Hapten wird üblicherweise ein Molekül bezeichnet, das nicht für sich allein immunogen ist, sondern das zu Immunisierungszwecken üblicherweise an einen Träger gebunden wird.

Unter dem Begriff "Antikörper" ist im Sinne dieser Erfindung ein Immunglobulin, zum Beispiel ein Immunglobulin der Klasse bzw. Subklasse IgA, IgD, IgE, IgG₁, IgG₂ₐ, IgG_{2b}, IgG₃, IgG₄, IgM, zu verstehen. Ein Antikörper weist mindestens eine Bindungsstelle (häufig Paratop genannt) für ein Epitop (häufig auch antigene Determinante genannt) auf einem Antigen oder Hapten auf. Ein solches Epitop ist zum Beispiel durch seine räumliche Struktur und/oder durch das Vorhandensein von polaren und/oder apolaren Gruppen gekennzeichnet. Die Bindungsstelle des Antikörpers ist komplementär zum Epitop. Die Antigen-Antikörper-Reaktion bzw. die Hapten-Antikörper-Reaktion funktioniert nach dem sogenannten "Schlüssel-Schloss-Prinzip" und ist in der Regel in einem hohen Grad spezifisch, d.h. die Antikörper vermögen kleine Abweichungen in der Primärstruktur, in der Ladung, in der räumlichen Konfiguration und der sterischen Anordnung des Antigens oder Haptens zu unterscheiden. Insbesondere die sogenannten "complementarity determining regions" des Antikörpers tragen zur Bindung des Antikörpers an das Antigen oder Hapten bei.

Unter dem Begriff "Antikörper" sind im Sinne dieser Erfindung aber nicht nur komplette Antikörper zu verstehen, sondern ausdrücklich auch Antikörperfragmente, wie z.B. Fab, Fv, F(ab')₂, Fab'; sowie auch chimäre, humanisierte, bi- oder oligospezifische, oder "single chain" Antikörper; des weiteren auch Aggregate, Polymere und Konjugate von Immunglobulinen und/oder deren Fragmente, sofern die Bindungseigenschaften an das Antigen oder Hapten erhalten sind. Antikörperfragmente lassen sich beispielsweise durch enzymatische Spaltung von Antikörpern mit Enzymen wie Pepsin oder Papain herstellen. Antikörperaggregate, -polymere und -konjugate können durch vielfältige Methoden generiert werden, z.B. durch Hitzebehandlung, Umsetzung mit Substanzen wie Glutaraldehyd, Reaktion mit Immunglobulin-bindenden Molekülen, Biotinylierung von Antikörpern und anschließende Reaktion mit Streptavidin oder Avidin, etc.

Bei einem Antikörper im Sinne dieser Erfindung kann es sich um einen monoklonalen oder um einen polyklonalen Antikörper handeln. Der Antikörper kann nach den üblichen Verfahren hergestellt worden sein, z.B. durch Immunisierung des Menschen oder eines Tieres, wie z.B. Maus, Ratte, Meerschweinchen, Kaninchen, Pferd, Esel, Schaf, Ziege, Huhn [s.a. Messerschmid (1996) BIOforum 11: 500-502], und anschließender Gewinnung des Antiserums; oder durch die Etablierung von Hybridomazellen und der anschließenden Reinigung der sekretierten Antikörper; oder durch Klonierung und Expression der Nukleotidsequenzen bzw. modifizierter Versionen davon, die die Aminosäuresequenzen kodieren, die für die Bindung des natürlichen Antikörpers an das Antigen und/oder Hapten verantwortlich sind.

Erfindungsgemäße Antikörper sind insbesondere solche Antikörper, die an die oben beschriebenen Proteine, Proteinkomplexe bzw. Peptide binden.

Durch die Bereitstellung der erfindungsgemäßen Antikörper ist es dem Fachmann nun möglich, z.B. durch Kompetitionsexperimente [s.a. Peters et al. (1985) Monoklonale Antikörper, Springer Verlag, Kapitel 12.2 "Epitop-Analyse"], andere spezifische Antikörper zu identifizieren, die an das Epitop eines erfindungsgemäßen Antikörpers binden. So lassen sich mittlerweile mit Hilfe von Phage Display Bibliotheken, über synthetische Peptiddatenbanken oder mittels "Recombinatorial Antibody Libraries" spezifische Bindungspartner selektieren [Larrick & Fry (1991) Human Antibodies and Hybridomas 2: 172-189].

Gegenstand dieser Erfindung ist auch ein erfindungsgemäßer Antikörper, der mit einer Festphase und/oder einer Komponente eines signalbildenden Systems assoziiert ist.

Der Begriff "Festphase" im Sinne dieser Erfindung beinhaltet einen Gegenstand, der aus porösem und/oder nicht porösem, in der Regel wasserunlöslichem Material besteht und die unterschiedlichsten Formen aufweisen kann, wie z.B. die von Gefäßen, Röhrchen, Mikrotitrationsplatten, Kugeln, Mikropartikeln, Stäbchen, Streifen, Filter- oder Chromatographiepapier, etc. In der Regel ist die Oberfläche der Festphase hydrophil oder kann hydrophil gemacht werden. Die Festphase kann aus den unterschiedlichsten Materialien bestehen wie z.B. aus anorganischen und/oder aus organischen Materialien, aus synthetischen, aus natürlich vorkommenden und/oder aus modifizierten natürlich vorkommenden Materialien. Beispiele für Festphasenmaterialien sind Polymere, wie z.B. Zellulose, Nitrozellulose, Zelluloseacetat, Polyvinylchlorid, Polyacrylamid, vernetzte Dextranmoleküle, Agarose, Polystyrol, Polyethylen, Polypropylen, Polymethacrylat oder Nylon; Keramik, Glas, Metalle, insbesondere Edelmetalle wie Gold und Silber; Magnetit; Mischungen oder Kombinationen derselben; etc. Auch Zellen, Liposomen oder Phospholipidvesikel sind vom Begriff Festphase miterfasst.

Die Festphase kann einen Überzug aus einer oder mehreren Schichten aufweisen z.B. aus Proteinen, Kohlehydraten, lipophilen Substanzen, Biopolymeren, organischen Polymeren oder Mischungen hiervon, um beispielsweise die unspezifische Bindung von Probenbestandteilen an die Festphase zu unterdrücken oder zu verhindern oder um beispielsweise Verbesserungen zu erreichen hinsichtlich der Suspensionsstabilität von partikulären Festphasen, der Lagerstabilität, der formgebenden Stabilität oder der Resistenz gegen UV-Licht, Mikroben oder sonstige zerstörend wirkende Agenzien.

Mikropartikel werden häufig als Festphase und/oder als Label genutzt. Unter dem Begriff "Mikropartikel" sind im Sinne dieser Erfindung Teilchen zu verstehen, die einen ungefähren Durchmesser von wenigstens 20 nm und nicht mehr als 20 µm aufweisen, üblicherweise zwischen 40 nm und 10 µm, bevorzugt zwischen 0,1 und 10 µm, besonders bevorzugt zwischen 0,1 und 5 µm, ganz besonders bevorzugt zwischen 0,15 und 2 µm. Die Mikropartikel können regelmäßig oder unregelmäßig geformt sein. Sie können Kugeln, Spheroide, Kugeln mit mehr oder weniger großen Kavitäten oder Poren darstellen. Die Mikropartikel können aus organischem, aus anorganischem Material oder aus einer Mischung oder Kombination von beiden bestehen. Sie können aus einem porösen oder nicht porösen, einem schwellfähigen oder nicht schwellfähigen Material bestehen. Prinzipiell können die Mikropartikel jegliche Dichte haben, bevorzugt sind jedoch Partikel mit einer Dichte, die der Dichte des Wassers nahe kommt wie etwa 0,7 bis etwa 1,5 g/ml. Die bevorzugten Mikropartikel sind in wässrigen Lösungen suspendierbar und möglichst lange suspensionsstabil. Sie mögen durchsichtig, teilweise durchsichtig oder undurchsichtig sein. Die Mikropartikel können aus mehreren Schichten bestehen wie beispielsweise die sogenannten "core-and-shell" Partikel mit einem Kern und einer oder mehreren umhüllenden Schichten. Der Begriff Mikropartikel umfasst beispielsweise Farbstoffkristalle, Metallsolen, Silica-Partikel, Glaspartikel, Magnetpartikel, Polymerteilchen, Öltropfen, Lipidpartikel, Dextran und Proteinaggregate. Bevorzugte Mikropartikel sind in wässrigen Lösungen suspendierbare und aus wasserunlöslichem Polymermaterial bestehende Partikel, insbesondere aus substituierten Polyethylenen. Ganz besonders bevorzugt sind Latexpartikel z.B. aus Polystyrol, Acrylsäurepolymeren, Methacrylsäurepolymeren, Acrylnitril-Polymeren, Acrylnitril-Butadien-Styrol, Polyvinylacetat-Acrylat, Polyvinylpyridin, Vinylchlorid-Acrylat. Von besonderem Interesse sind Latexpartikel mit reaktiven Gruppen an ihrer Oberfläche wie beispielsweise Carboxyl-, Amino- oder Aldehydgruppen, die eine kovalente Bindung z.B. von spezifischen Bindungspartnern an die Latexpartikel erlauben. Die Herstellung von Latexpartikeln ist beispielsweise in EP 0 080 614, EP 0 227 054 und EP 0 246 446 beschrieben.

Bei einem "signalbildenden System" kann es sich um eine oder mehrere Komponenten handeln, wobei es sich wenigstens bei einer Komponente um ein nachweisbares Label handelt. Als Label ist jedes Molekül zu verstehen, das selbst ein Signal produziert oder die Produktion eines Signals induzieren kann, wie z.B. eine fluoreszierende Substanz, eine radioaktive Substanz, ein Enzym oder eine chemilumineszierende Substanz. Das Signal kann beispielsweise anhand der Enzymaktivität, der Lumineszenz, der Lichtabsorption, der Lichtstreuung, der ausgestrahlten elektromagnetischen oder radioaktiven Strahlung oder einer chemischen Reaktion nachgewiesen oder gemessen werden.

Ein Label vermag selbst ein nachweisbares Signal zu erzeugen, so dass keine weiteren Komponenten notwendig sind. Viele organische Moleküle absorbieren ultraviolettes und sichtbares Licht, wodurch diese Moleküle in einen angeregten Energiezustand kommen können und die absorbierte Energie in Form von Licht einer anderen Wellenlänge als der des eingestrahlten Lichts abgeben. Wieder andere Label können direkt ein nachweisbares Signal erzeugen wie z.B. radioaktive Isotope oder Farbstoffe.

Wieder andere Label benötigen zur Signalerzeugung weitere Komponenten, d.h. das signalproduzierende System schließt in einem solchen Fall all die für die Signalbildung benötigten Komponenten mit ein wie z.B. Substrate, Coenzyme, Quencher, Beschleuniger, zusätzliche Enzyme, Substanzen, die mit Enzymprodukten reagieren, Katalysatoren, Aktivatoren, Cofaktoren, Inhibitoren, Ionen etc.

Geeignete Label sind beispielsweise Enzyme einschließlich Meerrettichperoxidase, alkalische Phosphatase, Glukose-6-Phosphatdehydrogenase, Alkoholdehydrogenase, Glucoseoxidase, β-Galactosidase, Luciferase, Urease und Acetylcholinesterase; Farbstoffe; fluoreszierende Substanzen einschließlich Fluoresceinisothiocyanat, Rhodamin, Phycoerythrin, Phycocyanin, Ethidiumbromid, 5-Dimethylaminonapthalen-1-sulfonylchlorid und fluoreszierende Chelate von seltenen Erden; chemilumineszierende Substanzen einschließlich Luminol, Isoluminol, Acridiniumverbindungen, Olefin, Enolether, Enamin, Arylvinylether, Dioxen, Arylimidazol, Lucigenin, Luciferin und Aequorin; Sensitizer einschließlich Eosin, 9,10-Dibromoanthracen, Methylen Blau, Porphyrin, Phthalocyanin, Chlorophyll, Rose Bengal; Coenzyme; Enzymsubstrate; radioaktive Isotope einschließlich ¹²⁵I, ¹³¹I, ¹⁴C, ³H, ³²P, ³³P, ³⁵S, ⁵¹Cr, ⁵⁹Fe, ⁵⁷Co und ⁷⁵Se; Partikel einschließlich magnetische Partikel oder Partikel, bevorzugt Latexpartikel, die selbst beispielsweise mit Farbstoffen, Sensitizern, fluoreszierenden Substanzen, chemilumineszierenden Substanzen, Isotopen oder anderen nachweisbaren Labeln markiert sein können; Solpartikel einschließlich Gold- oder Silbersolen; Liposomen oder Zellen, die selbst mit nachweisbaren Labeln markiert sein können; etc. [s.a. EP-A2-0 515 194; US 5,340,716; US 5,545,834; Bailey et al. (1987) J. Pharmaceutical & Biomedical Analysis 5: 649-658].

Ein signalbildendes System kann auch Komponenten umfassen, die bei räumlicher Nähe miteinander in eine nachweisbare Wechselwirkung treten können, z.B. in Form von Energiespendern und Energieempfängern wie beispielsweise Photosensitizer und chemolumineszierende Substanzen (EP-A2-0 515 194), Photosensitizer und Fluorophore (WO 95/06877), radioaktives Iod¹²⁵ und Fluorophore [Udenfriend et al. (1985) Proc. Natl. Acad. Sci. 82: 8672-8676], Fluorophore und Fluorophore [Mathis (1993) Clin. Chem. 39: 1953-1959] oder Fluorophore und Fluoreszenz-Quencher (US 3,996,345).

Unter einer Wechselwirkung zwischen den Komponenten ist die direkte Übertragung von Energie zwischen den Komponenten, z.B. durch Licht- oder Elektronenstrahlung sowie über kurzlebige reaktive chemische Moleküle, miteingeschlossen. Ferner umfasst sind hiervon auch Vorgänge, bei denen die Aktivität einer Komponente durch eine oder mehrere andere inhibiert oder verstärkt wird, beispielsweise die Hemmung oder Steigerung der Enzymaktivität oder die Hemmung, Steigerung oder Veränderung (z.B. Wellenlängenverschiebung, Polarisation) der von der beeinflussten Komponente ausgesendeten elektromagnetischen Strahlung. Die Wechselwirkung zwischen den Komponenten umfasst auch Enzymkaskaden. In diesem Fall sind die Komponenten Enzyme, von denen mindestens eines das Substrat für ein anderes liefert, so dass eine maximale oder minimale Reakionsgeschwindigkeit der gekoppelten Substratumsetzung resultiert.

Eine effektive Wechselwirkung zwischen den Komponenten findet in der Regel statt, wenn diese räumlich benachbart vorliegen, also z.B. innerhalb eines Abstandbereiches von wenigen µm, insbesondere innerhalb eines Abstandbereiches von unter 600 nm, bevorzugt unter 400 nm, ganz besonders bevorzugt von unter 200 nm.

Der Begriff "assoziiert" ist breit zu verstehen und umfasst beispielsweise eine kovalente und eine nicht-kovalente Bindung, eine direkte und eine indirekte Bindung, die Adsorption an eine Oberfläche und den Einschluß in eine Vertiefung oder einen Hohlraum, etc. Bei einer kovalenten Bindung sind die Antikörper oder Bindungspartner über eine chemische Bindung an die Festphase oder an das Label gebunden. Beispiele für eine nicht-kovalente Bindung sind die Oberflächenadsorption, der Einschluss in Hohlräume oder die Bindung von zwei spezifischen Bindungspartnem. Neben einer direkten Bindung an die Festphase oder das Label können die Antikörper oder Bindungspartner auch indirekt über spezifische Wechselwirkung mit anderen spezifischen Bindungspartnern an die Festphase oder das Label gebunden sein (s.a. EP-A2-0 411 945). Beispiele hierfür sind: biotinylierte Antikörper, die über labelgebundenes Avidin an das Label gebunden werden können oder ein Fluorescein-Antikörperkonjugat, das über festphasengebundene Anti-Fluorescein-Antikörper an die Festphase gebunden werden kann oder ein Antikörper, der über Immunglobulin-bindende Proteine an die Festphase oder das Label gebunden werden kann.

Ein weiterer Gegenstand dieser Erfindung sind erfindungsgemäße Antikörper, die als in vitro Diagnostikum oder als ein Bestandteil eines in vitro Diagnostikums verwendet werden.

Bei einem in vitro Diagnostikum wird der nachzuweisende Analyt, z.B. eine bestimmte P*l*GF-Form, in einer Probe außerhalb eines lebenden menschlichen oder tierischen Körpers nachgewiesen oder dessen Konzentration oder Menge bestimmt.

Unter einer "Probe" ist im Sinne der Erfindung das Material zu verstehen, das die nachzuweisende Substanz (Beispiele hierfür siehe EP-A2-0 515 194, "Analyt") vermutlich enthält. Der Begriff Probe umfasst beispielsweise biologische Flüssigkeiten oder Gewebe insbesondere von Menschen und Tieren wie Blut, Plasma, Serum, Sputum, Exudat, bronchoalveoläre Lavage, Lymphflüssigkeit, Synovialflüssigkeit, Samenflüssigkeit, Vaginalschleim, Feces, Urin, Liquor, Haare, Haut, Gewebeproben oder -schnitte. Ferner werden umfasst Zellkulturproben, pflanzliche Flüssigkeiten oder Gewebe, forensische Proben, Wasser- und Abwasserproben, Nahrungsmittel, Arzneimittel. Gegebenenfalls müssen die Proben vorbehandelt werden, um den Analyten für das Nachweisverfahren zugänglich zu machen oder um störende Probenbestandteile zu entfernen. Solche Vorbehandlung von Proben mag die Abtrennung und/oder Lyse von Zellen beinhalten, das Präzipitieren, die Hydrolyse oder die Denaturierung von Probenbestandteilen wie z.B. Proteinen, die Zentrifugation von Proben, das Behandeln der Probe mit organischen Lösungsmitteln wie z.B. Alkohole, insbesondere Methanol; das Behandeln der Probe mit Detergenzien. Häufig wird die Probe in ein anderes, meistens wässriges, Medium überführt welches mit dem Nachweisverfahren möglichst nicht interferieren soll.

Die erfindungsgemäßen Antikörper können in einem Verfahren zum quantitativen oder qualitativen Nachweis eines Analyten, bevorzugt bestimmte P*l*GF-Formen, insbesondere fP*l*GF, in einer Probe verwendet werden.

Bei einem quantitativen Nachweis wird die Menge, die Konzentration oder die Aktivität (z.B. Enzymaktivität) des Analyten in der Probe gemessen. Von dem Begriff des "quantitativen Nachweises" sind auch semiquantitative Methoden umfasst, die nur die ungefähre Menge, Konzentration oder Aktivität des Analyten in der Probe erfassen oder nur zu einer relativen Mengen-, Konzentrations- oder Aktivitätsangabe dienen können. Unter einem qualitativen Nachweis ist der Nachweis des Vorhandenseins des Analyten in der Probe überhaupt oder das Anzeigen, dass die Konzentration oder Aktivität des Analyten in der Probe unterhalb oder oberhalb eines bestimmten oder mehrerer bestimmter Schwellenwerte liegt, zu verstehen.

Die Erfindung betrifft somit auch Verfahren zum quantitativen oder qualitativen Nachweis eines Analyten, bevorzugt bestimmte P*l*GF-Formen, insbesondere fP*l*GF, in einer Probe und geeignete Reagenzien hierfür.

Zum Nachweis von Analyten werden häufig Bindungstests eingesetzt, bei denen durch eine spezifische Bindung von nachzuweisendem Analyt an analytspezifische Bindungspartner auf die Anwesenheit, Abwesenheit oder Menge des Analyten in einer Probe geschlossen werden kann. Immunoassays oder auch Verfahren, bei denen Oligo- oder Polynukleotide hybridisiert werden, sind Beispiele für Bindungstests.

Die sogenannten "heterogenen Bindungstests" sind durch einen oder mehrere Trennungsschritte und/oder Waschschritte gekennzeichnet. Die Trennung kann beispielsweise durch Immunfällung, Fällung mit Substanzen wie Polyethylenglykol oder Ammoniumsulfat, Filtration, magnetische Abtrennung, Anbindung an eine Festphase erfolgen. Bei heterogenen Bindungstests im Sandwichformat ist in der Regel einer der analytspezifischen Bindungspartner an eine Festphase gebunden und dient zur Abtrennung des Bindungskomplexes "Analyt/analytspezifischer Bindungspartner" von der flüssigen Phase, während der andere analytspezifische Bindungspartner zum Nachweis des Bindungskomplexes ein nachweisbares Label, z.B. ein Enzym, ein Fluoreszenz- oder Chemilumineszenzlabel etc. trägt. Man unterteilt diese Testverfahren weiter in sogenannte Einschritt-Sandwich-Tests, bei denen die beiden spezifischen Bindungspartner simultan mit der Probe inkubiert werden und in Zweischritt-Sandwich-Tests, bei denen die Probe zunächst mit dem Festphasenreagenz inkubiertwird und nach einem Trenn- und Waschschritt der festphasengebundene Bindungskomplex aus Analyt und analytspezifischer Bindungspartner mit dem Nachweisreagenz inkubiert wird.

Bei "homogenen Bindungstests" erfolgt keine Trennung zwischen freien und an den "Analyt/analytspezifischer Bindungspartner"-Komplex gebundenen Komponenten des signalbildenden Systems. Der Testansatz, welcher die analytspezifischen Bindungspartner, die signalbildenden Komponenten und die Probe enthält, wird nach oder sogar während der Bindungsreaktion ohne einen weiteren Trenn- und/oder Waschschritt gemessen und das entsprechende Messsignal bestimmt. Beispiele für homogene Immunoassays [s.a. Boguslaski & Li (1982) Applied Biochemistry and Biotechnology 7: 401-414] sind turbidimetrische oder nephelometrische Methoden, wobei die zum Nachweis verwendeten analytspezifischen Bindungspartner mit Latexpartikeln assoziiert sein können, wie z.B. EMIT^{®}-Tests; CEDIA^{®}-Tests; Fluoreszenz-Polarisations-Immunoassays; Luminescent Oxygen Channeling Immunoassays [LOCI^{®}, s. EP-A2-0 515 194; Ullman et al. (1994) Proc. Natl. Acad. Sci. 91: 5426-5430; Ullman et al. (1996) Clinical Chemistry 42: 1518-1526] etc. Bei einem homogenen Sandwich-Immunoassay, wie z.B. einem nephelometrischen Latextest, werden die Antikörperreagenzien mit der Probe zusammen inkubiert und die Messung des Signals während und/oder nach der Inkubation durchgeführt, ohne dass ein Trenn- oder Waschschritt vor der Messung durchgeführt wird. Mit anderen Worten: Es erfolgt keine Trennung des Antikörper-gebundenen Analyten vom freien Analyten oder von Antikörpern, die an keinen Analyten gebunden haben.

Homogene und heterogene Bindungstests können auch in Form eines sogenannten "Sandwichassays" durchgeführt werden. Hierbei wird der Analyt z.B. bei einem heterogenen Bindungstest von einem Festphasen-assoziierten, analytspezifischen Bindungspartner und einem analytspezifischen Bindungspartner, der mit einer Komponente eines signalbildenden System assoziiert ist, gebunden. Bei Sandwich-Immunoassays können Antikörper oder Antigene oder Haptene die analytspezifischen Bindungspartner bilden.

Eine weitere spezielle Ausführungsform eines heterogenen oder homogenen Bindungstests ist der "indirekte Immunoassay". Der Analyt ist in diesem Fall ein Antikörper. Einer der analytspezifischen Bindungspartner ist das Antigen oder z.B. ein erfindungsgemäßes Peptid oder ein modifiziertes Antigen des nachzuweisenden Antikörpers (= Analyt), und der andere analytspezifische Bindungspartner ist in der Regel ein Immunglobulin-bindendes Protein wie z.B. ein Antikörper, der den nachzuweisenden Antikörper (= Analyt) spezifisch zu binden vermag.

Bei einem homogenen oder heterogenen "kompetitiven Bindungstest" konkurrieren Proben-Analyt und Reagenz-Analyt um die Bindung an eine limitierte Anzahl von analytspezifischen Bindungspartnern. Der Reagenz-Analyt ist beispielsweise ein "modifizierter Analyt", wie z.B. ein gelabelter oder markierter Analyt, ein Analytteilstück wie z.B. die erfindungsgemäßen Peptide oder ein Analytanalogon. Beispiele zur Illustration des Prinzips: (i) Proben-Analyt konkurriert mit Reagenz-Analyt, der mit einer Komponente eines signalbildenden System assoziiert ist, um die Bindung an Festphasen-assoziierte, analytspezifische Bindungspartner oder (ii) Proben-Analyt konkurriert mit Festphasen-assoziiertem Analyt (= Reagenz-Analyt) um die Bindung an analytspezifische Bindungspartner, die mit einer Komponente eines signalbildenden Systems assoziiert sind.

Der Nachweis verschiedener P*l*GF-Formen mit den erfindungsgemäßen spezifischen Bindungspartneren, insbesondere Antikörpern, kann auch mit Verfahren erfolgen wie beispielsweise Western Blot, Dot Blot, Immunelektrophorese, Immunfixations-Elektrophorese, Elektroimmundiffusion, Immunpräzipitation, radiale Immundiffusion, Immunfixation, Immunchromatographie, Latex-Agglutination, turbidimetrischer oder nephelometrischer Test, homogener oder heterogener Bindungstest, Ein- oder Zweischritt-Test, Sandwich-Test, indirekter Test, kompetitiver Test, "point-of-care"-Tests, etc. Diese und andere Nachweisverfahren sind beispielsweise in "Labor und Diagnose", ed. L. Thomas, TH-Books Verlagsgesellschaft mbH, Frankfurt, 1998, Kapitel 60 oder in "Laboratory Techniques in Biochemistry and Molecular Biology - An Introduction to Radioimmunoassay and Related Techniques", ed. T. Chard, Elsevier, Amsterdam, 1987, beschrieben.

Der Begriff "point-of-care-Tests" oder "POC-Tests" schließt Tests ein, bei denen kein separates Analyse- oder Messgerät zur Testdurchführung oder Testauswertung benötigt wird. POC-Tests basieren in vielen Fällen auf immunchromatographischen Verfahren, Immunkomplexabtrennungen per Filtration und/oder Immunfixationstechniken. Die POC-Tests sind insbesondere für Messungen vor Ort, z.B. am Krankenbett oder daheim, für den Notarzt und/oder beim niedergelassenen Arzt und weniger für das Großlabor gedacht. POC-Tests können insbesondere auch von Personen, die keine eingehende medizinisch-technische Ausbildung und Erfahrung auf dem Gebiet der Laboratoriumsmedizin haben, durchgeführt werden. Unter dem Begriff "POC-Tests" sind im Sinne dieser Erfindung auch sogenannte Heimtests oder OTC-Tests zu verstehen, die von medizinischen Laien durchgeführt werden dürfen, so wie z.B. die diversen Schwangerschaftstests, die für den Heimgebrauch vertrieben werden. Andere POC-Tests betreffen beispielsweise den Nachweis von Herzinfarktmarkern, Drogen, Arzneimitteln, Infektions- und Entzündungsmarkern. Bei vielen POC-Testsn sind oder werden im Laufe der Testdurchführung spezifische Bindungspartner an oder auf Filter- oder Chromatographiestreifen oder- scheiben assoziiert. Eine positive oder negative Nachweisreaktion kann zum Beispiel mit dem Erscheinen oder Nichterscheinen einer Farbbande in einem bestimmten Testfeld verknüpft sein und/oder dem Erscheinen oder Nichterscheinen eines bestimmten Symbols, z.B. einem "+", einem "-" und/oder der Intensität des jeweiligen Meßsignals.

Ein POC-Test für bestimmte P*l*GF-Formen, insbesondere fP*l*GF, kann beispielsweise so aufgebaut sein: Probe und gelabelte spezifische Antikörper, die an die fP*l*GF-Form, aber nicht oder kaum an andere P*l*GF-Formen zu binden vermögen, werden auf einen Teststreifen aufgetragen. Geeignete Label sind z.B. gefärbte Latexpartikel, kolloidales Gold, Enzyme etc. Sofern die fP*l*GF-Form in der Probe enthalten ist, werden sich fP*l*GF / Antikörper-Komplexe ausbilden. Diese Komplexe bewegen sich z.B. mittels Kapillarkraft in Richtung auf einen Bereich, in dem andere spezifische Bindungspartner, insbesondere Antikörper, die an andere fP*l*GF-Epitope zu binden vermögen und die z.B. in Form einer Bande fixiert sind oder im Laufe des Testverfahrens fixiert werden (z.B. über eine Biotin-Avidin-Brücke). Die gelabelten fP*l*GF / Antikörper-Komplexe werden in diesem Bereich gebunden und bilden mit den fixierten spezifischen Bindungspartner, insbesondere Antikörpern, einen Sandwichkomplex aus. Die Intensität des Labelsignals ist hier proportional zur P*l*GF-Probenkonzentration. Bei einem kompetitiven POC-Testverfahren können beispielsweise Antikörper-Fragmente in einem Bereich des Teststreifens fixiert sein oder im Laufe des Testverfahrens fixiert werden. Dieser fixierte Antikörper würde mit der fP*l*GF-Form aus der Probe um die Bindung an gelabelte Anti-fP*l*GF-Antikörper kompetitieren. Alternativ können auch fixierte fP*l*GF-Antikörper und gelabeltes fP*l*GF-Protein bzw. die erfindungsgemäßen Peptide für den Aufbau eines kompetitiven fP*l*GF-Tests eingesetzt werden.

Eine besonders bevorzugte Ausführungsform des erfindungsgemäßen Verfahrens ist ein nephelometrischer oder ein turbidimetrischer Test, insbesondere ein solcher Test bei dem erfindungsgemäße Antikörper - bevorzugt an Mikropartikel (insbesondere an Latexpartikel) assoziiert - eingesetzt werden.

Ein anderer erfindungsgemäßer Gegenstand ist ein Testkit, der einen oder mehrere der erfindungsgemäßen Antikörper und/oder Peptide enthält. In einem solchen Kit sind üblicherweise alle oder nur einige Komponenten eines Tests in abgepackter Form enthalten. Die erfindungsgemäßen Antikörper und/oder Peptide können beispielsweise mit einer oder mehreren Festphasen und/oder einer oder mehreren Komponenten eines signalbildenden Systems assoziiert sein. Der Testkit kann beispielsweise Standards, Kontrollen sowie andere Reagenzien, wie z.B. Puffer, Waschlösungen, Messsignal-auslösende Lösungen und/oder Enzymsubstrat, Küvetten, Pipetten und/oder Testanweisungen enthalten. Ein besonders bevorzugter erfindungsgemäßer Testkit enthält an Latexpartikel assoziierte erfindungsgemäße Antikörper und/oder erfindungsgemäße Peptide.

Die erfindungsgemäßen Antikörper und Peptide lassen sich auch für die Affinitätschromatographie verwenden. Unter dem Begriff "Affinitätschromatographie" ist eine Methode zur Reinigung und Isolierung von Substanzen, insbesondere Biopolymeren, zu verstehen, die auf der Tatsache beruht, dass viele Substanzen mit für sie spezifischen Bindungspartnern eine selektive, nichtkovalente, reversible Bindung eingehen können. Das Prinzip des Verfahrens besteht darin, dass der spezifische Bindungspartner an eine unlösliche Matrix (z.B. poröse Gläser, Gele auf Agarose-, Cellulose-, Dextran-, Polymer- und Kieselgelbasis) in der Regel kovalent gebunden und in Kontakt mit einer die Substanz enthaltenden Probe gebracht wird. Die gesuchte Substanz wird wegen ihrer spezifischen Wechelswirkung mit dem Matrix-gebundenen spezifischen Bindungspartner immobilisiert und zurückgehalten, während alle anderen in der Probe enthaltenen Substanzen durch Elution abgetrennt werden. Anschließend wird die gesuchte Substanz mit einem geeigneten Elutionsmittel, das die nichtkovalente Bindung zwischen Substanz und spezifischem Bindungspartner aufhebt, von der Matrix abgelöst (s.a. E. Buddecke, 1989, Grundrisse der Biochemie, Walter de Gruyter, Kapitel 7 "Proteine").

Ein weiterer Gegenstand dieser Erfindung sind erfindungsgemäße Antikörper oder spezifische Bindungspartner die als ein Therapeutikum eingesetzt werden. Dies umfasst erfindungsgemäße Antikörper oder erfindungsgemäße Peptide in einem pharmazeutisch verträglichen, sterilen Injektionsmedium. Unter einem pharmazeutisch verträglichen, sterilen Injektionsmedium ist beispielsweise eine keimfreie, pyrogenfreie Lösung, z.B. Saline oder eine andere Elektrolytlösung, zu verstehen, wie sie üblicherweise zur intravenösen, intramuskulären, intraperitonealen oder subkutanen Verabreichung von Arzneimitteln, Impfstoffen oder Kontrastmitteln verwendet wird.

Ein weiterer Gegenstand dieser Erfindung ist die Verwendung der erfindungsgemäßen Antikörper als Diagnostikum oder als Bestandteil eines Diagnostikums.

Ein weiterer Gegenstand dieser Erfindung ist ein Verfahren zur Herstellung eines erfindungsgemäßen Antikörpers, welches dadurch gekennzeichnet ist, dass zur Immunisierung ein oder mehrere der oben beschriebenen Peptide eingesetzt werden.

Die erfindungsgemäßen Antikörper können auch hergestellt werden durch die Verwendung von natürlich vorkommendem und/oder rekombinantem P*l*GF und VEGF Proteinen, Proteinisoformen bzw. Fragmenten davon.

Ein weiterer Gegenstand der Erfindung ist die Verwendung von erfindungsgemäßen Proteinen, Proteinisoformen, Fragmenten, Abbauprodukten, Homologen, und Peptiden als Referenzmaterialien, Standards, Kalibratoren und Kontrollen. Referenzmaterialien sind Materialien oder Substanzen, die Eigenschaften besitzten, die in einer Art und Weise etabliert sind, dass Referenzmaterialien als Kalibratoren, Standards und Kontrollen eingesetzt werden. Zudem können Referenzmaterialien zur Überprüfung von Messverfahren und für die Zuordnung von bestimmten Werten, insbesondere "konventionell richtige Werte", verwendet werden. Die Verwendung von Referenzmaterialien als Kalibratoren, Standards und Kontrollen oder die Verwendung von Kalibratoren, Standards und Kontrollen, die sich auf Referenzmaterialien bzw. die dort angegebenen "konventionellen richtigen Werte" beziehen, ist für die Qualitätskontrolle und Qualitätssicherung wichtig.

Die als Immunisierungsantigen verwendeten Peptide können ungebunden und/oder trägergebunden zur Immunisierung verwendet werden.

Typische Träger sind beispielsweise Proteine, wie z.B. Ovalbumin, Albumin oder Keyhole Limpet Hämocyanin (KLH), oder Polymere, wie z.B. Polyethylenglykol, Polyacrylamid oder Poly-d-Glutamin-d-Lysin. Die Peptide können beispielsweise mit Hilfe von Carbodiimid oder Glutaraldehyd an diese Träger gebunden werden oder auch mittels eines heterobifunktionalen Reagenzes, welches auch als Abstandhalter ("Spacer") wirken kann, wie z.B. N-Maleimidobutyryloxysuccinimidester (GBMS). Weitere Beispiele und Kopplungsmethoden s.a. Wong, S. (1993) Chemistry of Protein Conjugation and Cross-Linking, CRC Press, Inc., Boca Raton.

Das Immunisierungsantigen kann beispielsweise in Phosphat-gepufferter Saline aufgenommen werden und mit Immun Easy Mouse Adjuvans versetzt werden. Diese Emulsion kann dann z.B. intradermal, intraperitoneal und/oder subkutan einem Tier, beispielsweise einem Kaninchen, einer Maus, einer Ratte, einem Meerschweinchen, einem Pferd, einem Esel, einem Schaf, einer Ziege, einem Huhn etc. appliziert werden. Booster-Injektionen, wobei das Immunisierungsantigen auch mit inkomplettem Freund'schen Adjuvans emulgiert sein kann, können helfen, die Immunantwort zu steigern.

Erfindungsgemäße polyklonale Antikörper können aus dem Antiserum der immunisierten Tiere gewonnen werden und können per Affinitätschromatographie über eine Matrix, an die beispielsweise die entsprechenden P*l*GF-Formen oder die als Immunisierungsantigen eingesetzten Peptide gebunden werden, weiter aufgereinigt werden.

Um erfindungsgemäße monoklonale Antikörper zu erzeugen, werden nach den allgemein bekannten Verfahren [s.a. Harlow & Lane (1988) Antibodies: A Laboratory Manual, Cold Spring Harbor Laboratory, Cold Spring Harbor; Peters et al. (1985) Monoklonale Antikörper: Herstellung und Charakterisierung, Springer Verlag] die Immunzellen immunisierter Tiere, wie z.B. einer Maus oder eines Kaninchens, mit Myelomzellen zum Erzeugen von Antikörper produzierenden Hybridomazellen verschmolzen und anschließend geeignete Klone isoliert und vereinzelt. Die Auswahl der die gewünschten monoklonalen Antikörper produzierenden Klone wird mit Hilfe spezifischer Screeningverfahren durchgeführt. Hierbei wird die Bindungsspezifität der in den Zellkulturüberstand abgegebenen Antikörper z.B. an das Immunisierungsantigen oder an einen etwaigen Träger des Immunisierungsantigens mittels Enzymimmunoassay, Radioimmunoassay und/oder Western Blot überprüft. Hybridome, die erfindungsgemäße Antikörper herstellen, werden klonal vermehrt. Die so gewonnenen Hybridomazelllinien stehen dann für eine dauerhafte Produktion von monoklonalen Antikörpern zur Verfügung. Größere Antikörpermengen lassen sich beispielsweise aus Zellkulturüberstand, insbesondere aus Fermentern oder Rollerkulturen sowie aus Aszites gewinnen.

Je nach gewünschtem Verwendungszweck ist es vorteilhaft nur Teile der Antikörper, wie z.B. Fab-, F(ab')₂-, oder Fab'-Fragmente einzusetzen. Diese können beispielsweise mit den dem Fachmann bekannten enzymatischen Spaltverfahren erzeugt werden [s.a. Harlow & Lane (1988) Antibodies: A Laboratory Manual, Cold Spring Harbor Laboratory, Cold Spring Harbor].

Die Antigenbindungsstellen eines Antikörpers befinden sich in den sogenannten variablen Domänen, die durch die V-Gene kodiert sind. Mit den bekannten gentechnischen Methoden [z.B. Sambrook et al. (1989) Molecular Cloning: A Laboratory Manual, Cold Spring Harbor Laboratory, Cold Spring Harbor, 2nd edition; McCafferty et al. (1990) Nature 348: 552-554] kann auch die entsprechende Nukleinsäuresequenz eines erfindungsgemäßen Antikörpers ermittelt werden sowie dadurch auch die entsprechende Aminosäuresequenz, sofern diese noch nicht per Aminosäuresequenzierung bereits bekannt war. Als Ausgangsmaterial für derartige Analysen können die Hybridomazellen bzw. die Antikörper produzierenden Immunzellen immunisierter Tiere eingesetzt werden.

In Kenntnis der Nuklein- und/oder Aminosäuresequenz können mit Hilfe üblicher gentechnologischer und molekularbiologischer Methoden [s.a. Johnson & Chiswell (1993) Current Opinion in Structural Biology 3: 564-571] dann humanisierte, chimäre, bi- oder oligospezifische Antikörper sowie von der "complementarity determining region" abgeleitete Peptide ("minimal recognition units"), single-chain Fragmente, und/oder funktionelle Fusionsprodukte, z.B. rekombinant hergestellte Antikörper-Enzym-Konstrukte, hergestellt werden [s.a. Larrick & Fry (1991) Human Antibodies and Hybridomas 2: 172-189; Kitano et al. (1986) Appl. Microbiol. Biotechnol. 24: 282-286; Thompson et al. (1986) J. Immunol. Methods 94: 7-12], die an die jeweiligen, spezifischen Epitope der P*l*GF-Formen, insbesondere an ein erfindungsgemäßes Peptid, binden. Mit solchen unter dem Begriff "Antikörper" eingeschlossenen Peptiden kann beispielsweise eine Verringerung der Immunogenität und/oder eine verstärkte Wirksamkeit bei Verabreichnung als Arzneimittel oder in vivo Diagnostikum erzielt werden, und/oder es ergeben sich Vorteile für den Einsatz als oder in einem in vitro Diagnostikum. Die Antikörper sind auch herstellbar, gegebenenfalls unter Zuhilfenahme gentechnologischer Methoden, in Pilzen wie z.B. Hefezellen [Fischer et al. (1999) Biol. Chem. 380: 825-839; Hiatt et al. (1992) Genetic Engineering 14: 49-64), pflanzlichen, tierischen und prokaryontischen Zellen (s.a. WO 95/25172) sowie isolierten menschlichen Zellen.

Ein weiterer Gegenstand dieser Erfindung sind auch Pilze, tierische, pflanzliche oder prokaryontische Zellen sowie isolierte menschliche Zellen, die einen erfindungsgemäßen Antikörper produzieren. Eine bevorzugte Ausführungsform dieser Erfindung umfasst Hybridomazelllinien, die die erfindungsgemäßen Antikörper produzieren.

Die im Folgenden beschriebenen Beispiele dienen zur exemplarischen Beleuchtung einzelner Aspekte dieser Erfindung.

### Beispiel 1: Herstellung fPlGF-spezifischer monoklonaler Antikörper

### a) Immunisierung von Mäusen

BALB/c Mäuse werden jeweils mit 20 µg Immunisierungsantigen (an KLH gebundenes Peptid mit der LRCTGCCGDENLHCVPVET (LAN 2)) in Immun Easy Mouse Adjuvans (Qiagen GmbH, Deutschland) intraperitoneal immunisiert. IAN 2 wurde mittels einer Festphasensynthese gemäß allgemein bekannter Methoden synthetisiert. Nach 4 und 8 Wochen erfolgte eine Booster-Injektion mit jeweils 20 µg Immunisierungsantigen ohne Adjuvans. Die letzten 3 Tage vor der Fusion werden die Mäuse intravenös mit jeweils 10 µg Immunisierungsantigen geboostert.

### b) Fusion

Nach dem Töten der Mäuse durch CO₂-Inhalation werden die Milzen entnommen und Einzelzellsuspensionen in serumfreiem Dulbeccos modifiziertem Eagle Medium (DMEM; PAN Biotech GmbH, Deutschland) hergestellt. Die Zellen werden zentrifugiert (652 × g) und 2 mal in DMEM gewaschen. Anschließend wird die Zellzahl mittels Trypanblau-Färbung bestimmt. Zu etwa 10⁸ Milzzellen werden 2 × 10⁷ Myelomzellen (Sp2/0) gegeben. Nach dem Zentrifugieren (360 × g) wird der Überstand verworfen, 1 ml Polyethylenglycol-Lösung (PEG 4000, Merck Eurolab GmbH, Deutschland; ca. 50 %ig in DMEM) auf das Zellpellet gegeben und nach Resuspension 1 Minute bei 37 °C inkubiert. Anschließend werden ca. 10 ml DMEM zugegeben und 2 bis 4 Minuten bei Raumtemperatur inkubiert. Die fusionierten Zellen werden abzentrifugiert (326 × g) und das Pellet in DMEM + 10 % fötalem Kälberserum (Bio Whittaker Europe, Belgien) + HAT-Medium (CC Pro GmbH, Deutschland) resuspendiert und in 24 Well-Zellkulturplatten (Corning Costar GmbH, Deutschland) abgefüllt. Die ungefähre Zellkonzentration beträgt 5 × 10⁴ bis 5 × 10⁶ Zellen pro Well.

Nach 2 bis 3 Wochen werden die entstandenen Zellkolonien (Hybride) entnommen und in neue Kulturplatten überführt.

### c) Screening

Die Spezifität der in die Zellkultur abgegebenen Antikörper wird in einem ersten Testschritt mit Hilfe von Mikrotiterplatten (Nunc GmbH & Co. KG, Deutschland), die mit einem Peptid mit der Aminosäuresequenz LRCTGCCGDENLHCVPVET beschichtet sind, getestet.

In jede Vertiefung der Mikrotiterplatte werden 100 µl Zellkulturüberstand (Verdünnung 1:2) pipettiert und 1 Stunde bei + 15 bis + 25 °C inkubiert. Nach zweimaligem Waschen der Platte mit Waschlösung POD (OSEW; Dade Behring Marburg GmbH, Deutschland) werden in jede Vertiefung 100 µl anti-Maus IgG/F(ab')₂-POD-Konjugat (Dade Behring Marburg GmbH, Deutschland) eingefüllt und 1 Stunde bei + 15 bis + 25 °C inkubiert. Nach weiterem zweimaligen Waschen der Platte wird in jede Vertiefung 100 µl Chromogen TMB-Lösung (Dade Behring Marburg GmbH, Deutschland) eingefüllt und weitere 30 Minuten bei + 15 bis + 25 °C inkubiert. Nach der Inkubation wird in jede Vertiefung 100 µl Stopplösung POD (Dade Behring Marburg GmbH, Deutschland) eingefüllt und die Mikrotitterplatte am BEP II (Behring-ELISA-Prozessor II, Dade Behring Marburg GmbH, Deutschland) bei 450 nm ausgewertet.

In einem zweiten Testschritt werden die Hybride nach Vereinzelung wie oben beschrieben noch einmal im gleichen Testformat überprüft.

### d) Klonierung

Einzelne Zellen von Hybriden, die fP*l*GF-spezifische Antikörper produzieren, werden mit einem Mikromanipulator (Leitz Messtechnik GmbH, Deutschland) kloniert. Kulturüberstände dieser Klone werden wie unter g) beschrieben gereinigt und wie unter e), h) und i) beschrieben näher charakterisiert.

### e) Bestimmung der Antikörpersubklasse

Die Subklasse der Antikörper gegen fP*l*GF wird mittels IsoStrip™-Mouse Monoclonal Antibody Isotyping Kit- der Fa. Boehringer Mannheim, Deutschland bestimmt.

### f) Produktion der Antikörper

Für die Produktion größerer Mengen Antikörper werden die entsprechenden Zellklone in Rollerflaschen (Coming Costar GmbH, Deutschland) überführt, und bis zum gewünschten Endvolumen bei + 37 °C expandiert. Danach wird die Rollerkultur-Suspension zur Entfernung der Zellen über 0,22 µm filtriert. Die jetzt zellfreie Antikörperlösung wird über Ultrafilter (Trenngrenze 30.000 Dalton) ankonzentriert und anschließend aufgereinigt.

### g) Reinigung der Antikörper

Die erhaltene Antikörperlösung wird mit 0,14 M Phosphatpuffer pH 8,6 umgepuffert und auf ein mit rProtein A Sepharose™ Fast Flow (Amersham Biosciences Europe GmbH, Deutschland) gefüllte Chromatographiesäule aufgetragen (pro 10 mg zu reinigendem Antikörper wird 1 ml rProtein A Sepharose™ Fast Flow eingesetzt). Alle nicht gebundenen Komponenten werden durch Waschen der Säule mit 0,14 M Phosphatpuffer pH 8,6 entfernt. Der gebundene Antikörper wird mit 0,1 M Zitronensäure pH 3,0 von der Säule eluiert und gegen 0,05 M Natriumacetat + 0,5 M NaCl + 0,05 M Tris + 0,01 % Natriumazid pH 7,0 dialysiert.

### h) Selektion geeigneter Antikörper für einen fPlGF Sandwich ELISA

Die Reaktion der monoklonalen anti-fP*l*GF-Antikörper mit dem fP*l*GF-spezifischen Epitop (beispielsweise das Peptid mit der Aminosäuresequenz LRCTGCCGDENLHCVPVET) wird untersucht:

### Reaktion mit fPlGF:

Als Festphase wird eine Mikrotiterplatte verwendet, die mit fP*l*GF beschichtet ist. Die antifP*l*GF-Antikörper aus Kulturüberständen werden daran inkubiert. Nach einem Waschschritt wird eine Bindung des Antikörpers an das P*l*GF durch ein Konjugat, bestehend aus polyklonalen anti-Maus-Antikörpern aus Kaninchen und dem Enzym Peroxidase mit anschließender Farbreaktion nachgewiesen.

### Reaktion mit einem sFlt-1 / PlGF-Komplex:

Als Festphase wird eine Mikrotiterplatte verwendet, die mit fP*l*GF beschichtet ist. sFlt-1 wird darin inkubiert. Das sFlt-1, das hierfür verwendet wird, ist "recombinant human VEGF R1 (Flt-1)/Fc Chimera" von R&D Systems (Katalognummer: 321-FL bzw. 321-FL/CF). Nach einem Waschschritt wird anti-fP*l*GF-Antikörper aus Kulturüberständen inkubiert. Nach einem Waschschritt wird keine oder nur eine geringe Bindung spezifischer anti-fP*l*GF-Antikörper nachweisbar sein, da die Bindungsstellen überwiegend durch sFlt-1 besetzt sind. fP*l*GFunspezifische Antikörper binden vermehrt auch an den sFlt-1 / P*l*GF-Komplex, d.h. gP*l*GF. Nach einem Waschritt wird eine Bindung dieser unspezifischer Antikörper durch ein Konjugat, bestehend aus polyklonalen anti-Maus-Antikörpern aus Kaninchen und dem Enzym Peroxidase mit anschließender Farbreaktion nachgewiesen.

Bei diesem Testsystem sind fP*l*GF-spezifische Antikörper diejenigen, die in der Reaktion mit einem sFlt-1 / P*l*GF-Komplex keine oder eine deutlich geringere Farbreaktion als bei der Reaktion mit einem fP*l*GF-spezifischen Peptid zeigen.

Entsprechend werden fP*l*GF-spezifische Antikörper selektiert. Die Eignung dieser Antikörper zur Verwendung als Festphasen Antikörper in einem Sandwich-ELISA mit fP*l*GFspezifischem Konjugatantikörper, der mit einem dem Fachmann bekannten Verfahren an Meerrettich-Peroxidase gekoppelt ist (z.B. Nakane-Konjugation), wird untersucht.

Die Überprüfung der Eignung erfolgt im Sandwich-ELISA, wie im Beispiel 2a) beschrieben. Die wesentlichen Entscheidungskriterien für die Eignung sind eine eindeutige Differenzierung zwischen fP*l*GF und sFlt-1 / P*l*GF Komplex. Weitere Kriterien sind die untere Nachweisgrenze, und die Linearität der Kalibrationskurve.

### Beispiel 2: Herstellung fPlGF-spezifischer monoklonaler Antikörper

### a) Immunisierung von Mäusen

BALB/c Mäuse werden jeweils mit 20 µg Immunisierungsantigen (an KLH gebundenes Peptid mit der GCCGDENLHK (IAN 2-1-1K)) in Immun Easy Mouse Adjuvans (Qiagen GmbH, Deutschland) intraperitoneal immunisiert. IAN 2-1-1K wurde mittels einer Festphasensynthese gemäß allgemein bekannter Methoden synthetisiert. Die Reinheit von IAN 2-1-1K wurde mittels Säulenchromatographie (Säule: Merck 250 x 4 mm) geprüft. Hierbei wurde als Puffer A: 0,1 % TFA/Wasser und als Puffer B: 0,08 % FFA/Acetonitril verwendet. Die Flußrate betrug 0.8 ml. Die Detektion erfolgte bei 220 nm. Des Weiteren wurde eine Matrix-Assisted Laser Desorption Ionisation Massenspektrometrie (MALDI-MS) durchgeführt. Der Hauptpeak lag bei 1075 m/z. Nach 4 und 8 Wochen erfolgte eine Booster-Injektion mit jeweils 20 µg Immunisierungsantigen ohne Adjuvans. Die letzten 3 Tage vor der Fusion werden die Mäuse intravenös mit jeweils 10 µg Immunisierungsantigen geboostert.

Die Fusion b), die Klonierung d), die Bestimmung der Antikörpersubklasse e), die Produktion der Antikörper f), die Reinigung der Antikörper g) und die Selektion geeigneter Antikörper für einen fP*l*GF Sandwich ELISA h) erfolgt wie in Beispiel 1. Für das Screening c) wird ebenfalls wie in Beispiel 1 verfahren. Mit der Ausnahme, dass die Spezifität der in die Zellkultur abgegebenen Antikörper in einem ersten Testschritt mit Hilfe von Mikrotiterplatten (Nunc GmbH & Co. KG, Deutschland), die mit einem Peptid mit der Aminosäuresequenz GCCGDENLHK (IAN: 2-1-1K) beschichtet sind, getestet werden.

### Beispiel 3: Nachweis von fPlGF in einer Probe

### a) Testverfahren A

Peroxidase-konjugierte anti-P*l*GF-Antikörper werden in Kombination mit einem erfindungsgemäßen monoklonalen anti-fP*l*GF-Antikörper in einem Enzymimmunoassay nach dem Sandwich-Prinzip eingesetzt.

Während der ersten Inkubation bindet sich das in der Probe enthaltene fP*l*GF - sofern vorhanden - an die gegen fP*l*GF gerichteten, erfindungsgemäßen Antikörper, die an der Oberfläche der Vertiefungen einer Mikrotitrationsplatte fixiert sind. Nach Auswaschen der Vertiefungen werden in einer zweiten Bindungsreaktion Peroxidase-konjugierte anti-P*l*GF-Antikörper verwendet, die gegen ein beliebiges Epitop - abgesehen vom Rezeptor-bindenden Epitop - von P*l*GF gerichtet sind. Mit Hilfe von spezifischen Peroxidase-konjugierten anti-P*l*GF-Antikörpem kann in diesem Testverfahren prinzipiell auch das Vorhandensein von bestimmten P*l*GF-Isoformen nachgewiesen werden. Beispielsweise kann ein P*l*GF-1 spezifischer Antikörper zum Nachweis von P*l*GF-1 verwendet werden, usw. Prinzipiell ist auch der Nachweis des Vorhandenseins verschiedener P*l*GF-Isoformen mit verschiedenen spezifischen Antikörpern möglich. Die überschüssigen Enzym-konjugierten Antikörper werden ausgewaschen. Anschließend wird die gebundene Enzym-Aktivität in den Vertiefungen bestimmt. Die enzymatische Umsetzung von Wasserstoffperoxid und Tetramethylbenzidin wird durch Zusatz von verdünnter Schwefelsäure unterbrochen. Die zur fP*l*GF-Antigenkonzentration proportionale Farbintensität wird photometrisch bei einer Wellenlänge von 450 nm bestimmt und entweder qualitativ über einen Cut Off ausgewertet oder anhand einer Kalibrationskurve aus Standards quantifiziert.

Ein solcher erfindungsgemäßer Sandwich-Immunoassay detektiert fP*l*GF spezifisch in nur einem Testverfahren.

### b) Testverfahren B

Peroxidase-konjugierte, erfindungsgemäße monoklonale anti-fP*l*GF-Antikörper werden in Kombination mit einem erfindungsgemäßen monoklonalen anti-fP*l*GF-Antikörper in einem Enzymimmunoassay nach dem Sandwich-Prinzip eingesetzt.

Wie in Testverfahren A bindet während der ersten Inkubation das in der Probe enthaltene fP*l*GF - sofern vorhanden - an die gegen fP*l*GF gerichteten, erfindungsgemäßen Antikörper, die an der Oberfläche der Vertiefungen einer Mikrotitrationsplatte fixiert sind. Nach Auswaschen der Vertiefungen werden in einer zweiten Bindungsreaktion Peroxidase-konjugierte anti-fP*l*GF-Antikörper verwendet. Die überschüssigen Enzym-konjugierten Antikörper werden ausgewaschen. Anschließend wird die gebundene Enzym-Aktivität in den Vertiefungen bestimmt. Die enzymatische Umsetzung von Wasserstoffperoxid und Tetramethylbenzidin wird durch Zusatz von verdünnter Schwefelsäure unterbrochen. Die zur fP*l*GF-Antigenkonzentration proportionale Farbintensität wird photometrisch bei einer Wellenlänge von 450 nm bestimmt und entweder qualitativ über einen Cut Off ausgewertet oder anhand einer Kalibrationskurve aus Standards quantifiziert.

Ein solcher erfindungsgemäßer Sandwich-Immunoassay detektiert fP*l*GF spezifisch in nur einem Testverfahren. Testverfahren B ist insbesondere deshalb bevorzugt, weil fP*l*GF im Allgemeinen als Homodimer, beispielsweise als fP*l*GF-1-Homodimer vorliegt, und an beiden Polen des Homodimers Rezeptor gebunden sein kann. Bei Testverfahren B wird ein Enzymimmunoassay nach dem Sandwich-Prinzip eingesetzt der einen immobilisierten anti-fP*l*GF-Antikörper und einen Peroxidase-konjugierten anti-fP*l*GF-Antikörper verwendet, so dass überwiegen fP*l*GF bestimmt wird, das an beiden Polen keinen Bindungspartner aufweist.

Entsprechend der beschriebenen Beispiele und Testverfahren können analog auch die Spezifitäten der anderen erfindungsgemäßen Bindungspartner, insbesondere der Antikörper, ermittelt und diagnostisch eingesetzt werden.

### Beispiel 4: Herstellung weiterer fPlGF-spezifischer monoklonaler Antikörper

Die folgenden monoklonalen Antikörper wurden entsprechend Beispiel 1 hergestellt, selektiert und hinsichtlich ihrer Erkennung von freien und gebundenen P*l*GF-Formen untersucht. Zur Immunisierung wurden die in der nachfolgenden Tabelle angegebenen Immunisierungsantigene eingesetzt. Bei dem Antikörper "MAB264" von R&D Systems handelt es sich um einen Antikörper aus dem Stand der Technik, der nicht hier hergestellt wurde.

| **Monoklonaler Antikörper** | **Immunisierungsantigen** | **Spezifität** |
|---|---|---|
| 05-54/04 | rekombinantes humanes | freies PlGF |
| 05-64/026 | P*l*GF; | |
| 05-81-05 | R&D Systems, Katalog- | |
| 05-81-010 | Nr: 264-PG/CF | |
| (Gruppe 1) | | |
| 05-61/016 | rekombinantes humanes | freies und gebundenes PlGF |
| 05-63/020 | P*l*GF; R&D Systems, Katalog- | |
| (Vergleichsantikörper) | Nr: 264-PG/CF | |
| 05-164/012 | IAN 3-3-1 | freies PlGF |
| 05-164/038 | (RSGDRPSYVELT) | |
| 05-164/042 | | |
| 05-164/054 | | |
| (Gruppe 2) | | |
| 05-121/06 | IAN 1-1-2 | freies PlGF |
| 05-120/03 | (VVPFQEVWGRSY) | |
| (Gruppe 3) | | |
| R&D Systems | | freies und gebundenes PlGF |
| MAB264 (Vergleichsantikörper, Stand der Technik) | | |

Die Zellkulturen, welche die in der Tabelle angegebenen erfindungsgemäßen monoklonalen Antikörper oder die Vergleichsantikörper produzieren, wurden bei der DSMZ (Deutsche Sammlung von Mikroorganismen und Zellkulturen GmbH), Mascheroder Weg 1b, 38124 Braunschweig, Bundesrepublik Deutschland, gemäß Budapester Vertrag unter den nachfolgend angegebenen, von der Internationalen Hinterlegungsstelle zugeteilten Eingangsnummern hinterlegt:

| | |
|---|---|
| Zellkultur 2005-81-05 | = DSM ACC2764 |
| Zellkultur 2005-81-010 | = DSM ACC2765 |
| Zellkultur 2005-64/026 | = DSM ACC2766 |
| Zellkultur 2005-63/020 | = DSM ACC2767 |
| Zellkultur 2005-61/016 | = DSM ACC2768 |
| Zellkultur 2005-54/04 | = DSM ACC2769 |
| Zellkultur 2005-164/054 | = DSM ACC2770 |
| Zellkultur 2005-164/042 | = DSM ACC2771 |
| Zellkultur 2005-164/038 | = DSM ACC2772 |
| Zellkultur 2005-164/012 | = DSM ACC2773 |
| Zellkultur 2005-121/06 | = DSM ACC2774 |
| Zellkultur 2005-120/03 | = DSM ACC2775 |

Hinterlegungsdatum ist bei allen o.g. Zellkulturen der 23.03.2006.

### Beispiel 5: Reaktivität der erfindungsgemäßen Antikörper und der Vergleichsantikörper mit rekombinantem (freiem) PlGF

Mikrotitrationsplatten (Fa. Nunc, Typ B), wurden mit polyklonalem Antikörper gegen Maus-IgG /F(ab)₂ (Dade Behring Marburg GmbH, Deutschland) beschichtet; Beschichtungskonzentration 10 µg/ml ≈1,5 µg/Vertiefung.

In die Vertiefungen der Mikrotitrationsplatte wurden 100 µl der zu untersuchenden monoklonalen Antikörper in einer Konzentration von a) 1,0 µg/ml oder b) 0,1 µg/ml pipettiert und 1 Stunde bei +15 bis +25°C inkubiert. Nach dreimaligem Waschen der Mikrotitrationsplatte mit Waschlösung POD (Produkt-Nr.: OSEW; Fa. Dade Behring, Marburg, Deutschland) wurden in jede Vertiefung 100 µl einer Lösung von rekombinantem P*l*GF (R&D Systems, Katalog Nr: 264-PG/CF) in einer Konzentration von 0,1 µg/ml gegeben und 1 Stunde bei +15 bis +25°C inkubiert. Nach dreimaligem Waschen der Mikrotitrationsplatte mit Waschlösung POD wurden in jede Vertiefung 100 µl Anti-Human-PlGF-POD-Konjugat (hergestellt mittels eines konventionellen Konjugationsverfahrens aus "human P*l*GF Affinity Purified polyclonal Antibody", Prod. No.: AF-264-PB / R&D Systems) eingefüllt und 1 Stunde bei +15 bis +25°C inkubiert. Nach weiterem dreimaligen Waschen der Mikrotitrationsplatte wurden in jede Vertiefung 100 µl Chromogen TMB-Lösung (Produkt-Nr.: OUVF, Fa. Dade Behring Marburg GmbH, Deutschland) eingefüllt und weitere 30 Minuten bei +15 bis +25°C inkubiert. Nach der Inkubation wurden in jede Vertiefung 100 µl Stopplösung POD (Produkt-Nr.: OSFA, Fa. Dade Behring Marburg GmbH, Deutschland) eingefüllt und die Mikrotitrationsplatte am BEP II (Fa. Dade Behring Marburg GmbH, Deutschland) bei 450 nm ausgewertet. Die Ergebnisse sind in Tabelle 1 aufgelistet.

**Tabelle 1: Bestimmung der Reaktivität mit PlGF durch Auswertung der Mikrotitrationsplatten am BEP II bei 450 nm.**

| **Antikörperkonzentration [µg/ml]** | **Extinktion bei 450 nm** | | | | | | | | | | | |
|---|---|---|---|---|---|---|---|---|---|---|---|---|
| | **Erfindungsgemäße Antikörper, Gruppe 1** | | | | **Erfindungsgemäße Antikörper, Gruppe 2** | | | | **Erfindungsgemäßer Antikörper, Gruppe 3** | **Vergleichsantikörper** | | **Antikörper, gemäß dem Stand der Technik** |
| | **05.54/ 04** | **05-64/ 026** | **05-81-05** | **05-81-010** | **05-164/ 012** | **05-164/ 038** | **05-164/ 042** | **05-164/ 054** | **05-121/06** | **05-61/016** | **05-63/020** | **R&D Systems: MAB264** |
| **1,0** | 2,5 | 2,5 | 2,5 | 2,5 | 0,323 | 0,370 | 0,389 | 0,396 | 1,343 | 2,5 | 2,5 | 2,5 |
| **0,1** | 2,5 | 2,073 | 0,659 | 0,609 | 0,099 | 0,145 | 0,138 | 0,126 | 0,177 | 2,5 | 2,5 | 1,896 |

### Beispiel 6: Reaktivität der erfindungsgemäßen Antikörper, der Vergleichsantikörper und der Antikörper aus dem Stand der Technik mit PlGF/sFlt-1 Komplex (gPlGF)

Mikrotitrationsplatten (Fa. Nunc, Typ B), wurden mit polyklonalem Antikörper gegen human IgG/Fc (Fa. Dade Behring Marburg GmbH, Deutschland) beschichtet. Beschichtungskonzentration 2,5 µg/ml ≈ 0,376 µg/Vertiefung.

In die Vertiefungen der Mikrotitrationsplatte wurden je 100 µl einer Lösung von rekombinanter humaner VEGF R1 (Flt-1)/Fc Chimäre: R&D Systems, Katalog-Nr: 321-FL/CF) in einer Konzentration von 1 µg/ml gegeben und 1 Stunde bei +15 bis +25°C inkubiert. Nach dreimaligem Waschen der Mikrotitrationsplatte mit Waschlösung POD (s. Beispiel 5) wurden zur Herstellung des PlGF/sFlt-1-Komplexes in jede Vertiefung 100 µl einer Lösung von rekombinantem P*l*GF (s. Beispiel 5) in einer Konzentration von 1 µg/ml gegeben und 1 Stunde bei +15 bis +25°C inkubiert. Nach dreimaligem Waschen der Mikrotitrationsplatte mit Waschlösung POD wurden in jede Vertiefung 100 µl der zu untersuchenden monoklonalen Antikörper mit einer Konzentration von a) 1 µg/ml oder b) 0,1 µg/ml pipettiert und 1 Stunde bei +15 bis +25°C inkubiert. Nach dreimaligem Waschen der Mikrotitrationsplatte mit Waschlösung POD wurden in jede Vertiefung 100 µl anti-Maus IgG/F(ab)₂-POD-Konjugat (Fa. Dade Behring Marburg GmbH, Deutschland) eingefüllt und 1 Stunde bei +15 bis +25°C inkubiert. Nach weiterem dreimaligen Waschen der Mikrotitrationsplatte wurden in jede Vertiefung 100 µl Chromogen TMB-Lösung (s. Beispiel 5) eingefüllt und weitere 30 Minuten bei +15 bis +25°C inkubiert. Nach der Inkubation wurden in jede Vertiefung 100 µl Stopplösung POD (s. Beispiel 5) eingefüllt und die Mikrotitrationsplatte am BEP II (s. Beispiel 5) bei 450 nm ausgewertet. Die Ergebnisse sind in Tabelle 2 aufgelistet.

**Tabelle 2: Bestimmung der Reaktivität mit PlGF/sFlt-1Komplex durch Auswertung der Mikrotitrationsplatten am BEP II bei 450 nm.**

| **Antikörper-Konzentration [µg/ml]** | **Extinktion bei 450 nm** | | | | | | | | | | | |
|---|---|---|---|---|---|---|---|---|---|---|---|---|
| | **Erfindungsgemäße Antikörper, Gruppe 1** | | | | **Erfindungsgemäße Antikörper, Gruppe 2** | | | | **Erfindungs gemäß Antikörper, Gruppe 3** | **Vergleichsantikörper** | | **Antikörper, gemäß dem Stand der Technik** |
| | **05-54/04** | **05-64/026** | **05-81-05** | **05-81-010** | **05-164/012** | **05-164/038** | **05-164/042** | **05-164/054** | **05-121/06** | **05-61/016** | **05-63/020** | **R&D Systems: MAB264** |
| **1,0** | 0,036 | 0,032 | 0.043 | 0,045 | 0.027 | 0,026 | 0,029 | 0,023 | 0,028 | 2,500 | 2,500 | 0,691 |
| **0,1** | 0,035 | 0.028 | 0,026 | 0,026 | 0,022 | 0,023 | 0,023 | 0,023 | 0,028 | 0,692 | 0,750 | 0,217 |

Die erfindungsgemäßen Antikörper zeigen keine Reaktion mit dem gebildeten P*l*GF/sFlt-1Komplex. Wobei die Vergleichsantikörper und der Antikörper aus dem Stand der Technik eine deutliche Reaktion zeigen.

### Beispiel 7: Epitop-Kartierung

Scans überlappender Peptide, die aus der Sequenz des humanen PlGF abgeleitet wurden (13-mere Peptide, 11 Aminosäuren überlappend), wurden mit Hilfe der SPOT-Synthese-Technologie hergestellt. Die Verfahren sind beschrieben in: Wenschuh, H. et al. (2000) "Coherent membrane supports for parallel microsynthesis and screening of bioactive peptides", Biopolymers (Peptide Science), 55:188-206. Die Peptide wurden in definierter Anordnung (als "Peptid-Array") schrittweise an Zellulosemembranen synthetisiert, so daß sie kovalent an die Zellulosemembran gekoppelt vorlagen. Bindungstests zur Überprüfung der Immunreaktivität der Peptide wurden unmittelbar auf den Arrays durchgeführt. Das Inkubationsprotokoll dafür lautete wie folgt:
- Äquilibrierung in TBS-Puffer, pH 8.0
- 2 h Blockierungspuffer, pH 8.0
- 2 h Antikörperinkubation (3 µg/ml) in Blockierungspuffer, pH 8.0
- Waschen mit TBS (0.05% Tween20)
- 2 h Inkubation mit anti-Maus-IgG-POD in Blockierungspuffer, pH 8.0
- 3 x 5 min Waschen mit TBS (0.05% Tween20)
- Detektion mittels Chemolumineszenz (Lumi-Imager), Roche Diagnostics)

Ergebnisse: Die beiden erfindungsgemäßen Antikörper 05-81-05 und 05-81-010 aus der Gruppe 1 reagieren mit den folgenden Sequenzabschnitten:
**1. EKMKPERCGDAVP**
**2. MKPERCGDAVPRR**

Die erkannte Sequenz ist identisch mit der C-terminalen Domäne des P*l*GF-1.

### Zusätzliche Testungen mit zu untersuchenden monoklonalen Antikörpern auf der Festphase:

In den folgenden Beispielen 8 und 9 wurden ausschließlich die höheraffinen Antikörper der Gruppe 1 im Vergleich zum Stand der Technik und den Vergleichsantikörpern untersucht.

### Beispiel 8: Reaktion mit PlGF

Mikrotitrationsplatten (s. Beispiel 5), wurden mit den erfindungsgemäßen monoklonalen Antikörpern, mit den Vergleichsantikörpern und mit monoklonalen Antikörpern aus dem Stand der Technik beschichtet. Beschichtungskonzentration 3 µg/ml ≈ 0,45 µg/Vertiefung.

In die Vertiefungen der Mikrotitrationsplatte wurden 100 µl einer geometrischen Verdünnungsreihe beginnend mit 50 ng/ml rekombinantem P*l*GF (s. Beispiel 5) pipettiert und 1 Stunde bei +15 bis +25°C inkubiert. Nach dreimaligen Waschen der Platte mit Waschlösung POD (s. Beispiel 5) wurden in jede Vertiefung 100 µl Anti-Human-P*l*GF-POD-Konjugat (s. Beispiel 5) eingefüllt und 1,5 Stunde bei +15 bis +25°C inkubiert. Nach weiterem dreimaligen Waschen der Platte wurde in jede Vertiefung 100 µl Chromogen TMB-Lösung (s. Beispiel 5) eingefüllt und weitere 30 Minuten bei +15 bis +25°C inkubiert. Nach der Inkubation wurde in jede Vertiefung 100 µl Stopplösung POD (s. Beispiel 5) eingefüllt und die Mikrotitrationsplatte am BEP II (s. Beispiel 5) bei 450 nm ausgewertet. Die Ergebnisse sind in Tabelle 3 aufgelistet.

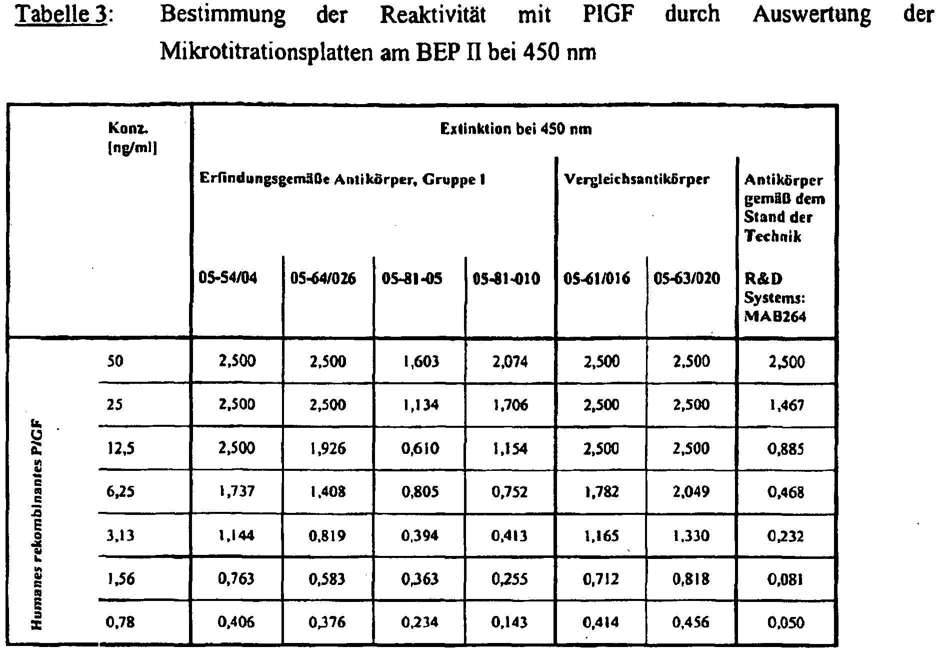

### Beispiel 9: Reaktivität der erfindungsgemäßen Antikörper, der Vergleichsantikörper und der Antikörper aus dem Stand der Technik mit PlGF/sFlt-1 Komplex (gPlGF)

Mikrotitrationsplatten (s. Beispiel 5), wurden mit den erfindungsgemäßen monoklonalen Antikörpern, mit Vergleichsantikörpern und mit monoklonalen Antikörpern aus dem Stand der Technik beschichtet. Beschichtungskonzentration 3 µg/ml ≈ 0,45 µg/Vertiefung.

In einem Reaktionsgefäß wurde eine geometrische Verdünnungsreihe beginnend mit 25 ng/ml rekombinantem P*l*GF hergestellt. Zu jeder Verdünnung wurde rekombinanter humaner VEGF R1 (Flt-1)/Fc Chimäre (s. Beispiel 6) in einer Konzentration von 400ng/ml zudosiert und 1 Stunde bei +15 bis + 25°C inkubiert. Anschließend wurden jeweils 100 µl in die Vertiefungen der Mikrotitrationsplatte pipettiert und 1 Stunde bei +15 bis +25°C inkubiert. Nach viermaligen Waschen der Mikrotitrationsplatte mit Waschlösung POD (s. Beispiel 5) wurden in jede Vertiefung 100 µl Anti-Human-VEGF R1-POD-Konjugat (Fa. R&D Systems, Part 891096 aus Quantikine Human VEGF R1 Immunoassay; DVR100B) eingefüllt und 1 Stunde bei +15 bis +25°C inkubiert. Nach weiterem dreimaligen Waschen der Mikrotitrationsplatte wurde in jede Vertiefung 100 µl Chromogen TMB-Lösung (s. Beispiel 5) eingefüllt und weitere 30 Minuten bei +15 bis +25°C inkubiert. Nach der Inkubation wurde in jede Vertiefung 100 µl Stopplösung POD (s. Beispiel 5) eingefüllt und die Mikrotitrationsplatte am BEP II (s. Beispiel 5) bei 450 nm ausgewertet. Die Ergebnisse sind in Tabelle 4 aufgelistet.

**Tabelle 4: Bestimmung der Reaktivität mit PlGF/sFlt-1Komplex durch Auswertung der Mikrotitrationsplatten am BEP II bei 450 nm.**

| **Konzentration [ng/ml]** | | **Extinktion bei 450 nm** | | | | | | |
|---|---|---|---|---|---|---|---|---|
| | | **Erfindungsgemäße Antikörper, Gruppe 1** | | | | **Vergleichsantikörper** | | **Antikörper gemäß dem Stand der Technik** |
| **P*l*GF** | **VEGF R1** | **05-54/04** | **05-64/026** | **05-81-05** | **05-81-010** | **05-61/016** | **05-63/020** | **R&D Systems: MAB264** |
| **25** | **400** | 0,079 | 0,143 | 0,260 | 0,204 | 1,772 | 2,500 | 2,188 |
| **12,5** | **400** | 0,047 | 0,099 | 0,264 | 0,105 | 1,642 | 2,045 | 1,611 |
| **6,25** | **400** | 0,038 | 0.071 | 0,116 | 0,088 | 0,936 | 1,530 | 0.883 |
| **3,13** | **400** | 0,04 | 0,069 | 0,072 | 0,059 | 0,477 | 0.868 | 0,529 |
| **1,56** | **400** | 0,037 | 0,068 | 0,046 | 0,049 | 0.278 | 0.404 | 0,272 |
| **0,78** | **400** | 0,038 | 0,063 | 0,042 | 0,045 | 0,177 | 0,282 | 0,146 |
| **0,39** | **400** | 0,047 | 0,073 | 0,043 | 0,049 | 0,116 | 0,189 | 0,094 |

Die erfindungsgemäßen Antikörper erkennen den P*l*GF/sFlt-1Komplex nicht bzw. nur sehr schwach. Sie sind spezifisch für freies P*l*GF, während die Vergleichsantikörper und der Antikörper aus dem Stand der Technik deutliche Reaktionen zeigen, also nicht für freies P*l*GF spezifisch sind.

Die vorliegende Erfindung wird ausdrücklich auch durch die folgenden Patentansprüche beschrieben, ohne die Erfindung jedoch darauf zu beschränken.

## Patentansprüche

1. Immunologisch aktives Peptid, welches die Sequenzinformation einer primären PlGF-Isoform besitzt, bestehend aus einer der folgenden Aminosäuresequenzen:
IAN 1: SAGNGSSEVE VVPFQEVWGR SYCRALERLV
IAN 3: VETANVTMQL LKIRSGDRP SYVELTFSQH
oder aus einem Teil der Peptide IAN 1 oder IAN 3, worin der Teil mindestens eine der Aminosäuresequenzen VVPFQEVWGRSY (IAN: 1-1-2) oder RSGDRPSYVELT (IAN: 3-3-1) enthält.

2. Monoklonaler Antikörper 05-81-05, der von der Zellinie mit der Bezeichnung 2005-81-05 produziert wird, die bei der DSMZ unter der Eingangsnummer DSM ACC2764 hinterlegt wurde.

3. Monoklonaler Antikörper 05-81-010, der von der Zellinie mit der Bezeichnung 2005-81-010 produziert wird, die bei der DSMZ unter der Eingangsnummer DSM ACC2765 hinterlegt wurde.

4. Monoklonaler Antikörper 05-64/026, der von der Zellinie mit der Bezeichnung 2005-64/026 produziert wird, die bei der DSMZ unter der Eingangsnummer DSM ACC2766 hinterlegt wurde.

5. Monoklonaler Antikörper 05-54/04, der von der Zellinie mit der Bezeichnung 2005-54/04 produziert wird, die bei der DSMZ unter der Eingangsnummer DSM ACC2769 hinterlegt wurde.

6. Monoklonaler Antikörper 05-164/054, der von der Zellinie mit der Bezeichnung 2005-164/054 produziert wird, die bei der DSMZ unter der Eingangsnummer DSM ACC2770 hinterlegt wurde.

7. Monoklonaler Antikörper 05-164/042, der von der Zellinie mit der Bezeichnung 2005-164/042 produziert wird, die bei der DSMZ unter der Eingangsnummer DSM ACC2771 hinterlegt wurde.

8. Monoklonaler Antikörper 05-164/038, der von der Zellinie mit der Bezeichnung 2005-164/038 produziert wird, die bei der DSMZ unter der Eingangsnummer DSM ACC2772 hinterlegt wurde.

9. Monoklonaler Antikörper 05-164/012, der von der Zellinie mit der Bezeichnung 2005-164/012 produziert wird, die bei der DSMZ unter der Eingangsnummer DSM ACC2773 hinterlegt wurde.

10. Monoklonaler Antikörper 05-121/06, der von der Zellinie mit der Bezeichnung 2005-121/06 produziert wird, die bei der DSMZ unter der Eingangsnummer DSM ACC2774 hinterlegt wurde.

11. Monoklonaler Antikörper 05-120/03, der von der Zellinie mit der Bezeichnung 2005-120/03 produziert wird, die bei der DSMZ unter der Eingangsnummer DSM ACC2775 hinterlegt wurde.

12. Spezifischer Antikörper, welcher mit einem oder mehreren der monoklonalen Antikörper gemäß einem oder mehreren der Ansprüche 2, 3 und 6 bis 11 um die Bindung an freies P*l*GF konkurriert.

13. Spezifischer Antikörper, welcher an ein Epitop bindet, enthalten in der folgenden Aminosäuresequenz: VVPFQEVWGRSY.

14. Peptide gemäß Anspruch 1 zur Verwendung als Immunisierungsantigene zur Herstellung fP*l*GF-spezifischer Antikörper.

15. Immunoassay zum Nachweis von P*l*GF in einer Probe, **dadurch gekennzeichnet, daß** ein spezifischer Antikörper gemäß einem oder mehreren der Ansprüche 2 bis 13 mit einer Probe in Kontakt gebracht und die Bildung eines Immunkomplexes unter Beteiligung des P*l*GF qualitativ oder quantitativ bestimmt wird.

16. Testkit zur Durchführung eines Immunoassays gemäß Anspruch 15 enthaltend einen Antikörper gemäß einem oder mehreren der Ansprüche 2 bis 13.

## Claims

1. Immunologically active peptide which possesses the sequence information of a primary P*l*GF isoform, consisting of one of the following amino acid sequences:
IAN 1: SAGNGSSEVEVVPFQEVWGRSYCRALERLV
IAN 3: VETANVTMQLLKIRSGDRPSYVELTFSQH
or of a part of the peptides IAN 1 or IAN 3, in which the part contains at least one of the amino acid sequences VVPFQEVWGRSY (IAN: 1-1-2) or RSGDRPSYVELT (IAN: 3-3-1).

2. Monoclonal antibody 05-81-05, which is produced by the cell line with the designation 2005-81-05 which was deposited at the DSMZ under the entry number DSM ACC2764.

3. Monoclonal antibody 05-81-010, which is produced by the cell line with the designation 2005-81-010 which was deposited at the DSMZ under the entry number DSM ACC2765.

4. Monoclonal antibody 05-64/026, which is produced by the cell line with the designation 200564/026 which was deposited at the DSMZ under the entry number DSM ACC2766.

5. Monoclonal antibody 05-54/04, which is produced by the cell line with the designation 200554/04 which was deposited at the DSMZ under the entry number DSM ACC2769.

6. Monoclonal antibody 05-164/054, which is produced by the cell line with the designation 2005164/054 which was deposited at the DSMZ under the entry number DSM ACC2770.

7. Monoclonal antibody 05-164/042, which is produced by the cell line with the designation 2005164/042 which was deposited at the DSMZ under the entry number DSM ACC2771.

8. Monoclonal antibody 05-164/038, which is produced by the cell line with the designation 2005164/038 which was deposited at the DSMZ under the entry number DSM ACC2772.

9. Monoclonal antibody 05-164/012, which is produced by the cell line with the designation 2005164/012 which was deposited at the DSMZ under the entry number DSM ACC2773.

10. Monoclonal antibody 05-121/06, which is produced by the cell line with the designation 2005121/06 which was deposited at the DSMZ under the entry number DSM ACC2774.

11. Monoclonal antibody 05-120/03, which is produced by the cell line with the designation 2005120/03 which was deposited at the DSMZ under the entry number DSM ACC2775.

12. Specific antibody, which competes for binding to free P*l*GF with one or more of the monoclonal antibodies according to one or more of Claims 2, 3 and 6 to 11.

13. Specific antibody, which binds to an epitope contained in the following amino acid sequence: VVPFQEVWGRSY.

14. Peptides according to Claim 1 for use as immunization antigens for the production of fP*1*GF-specific antibodies.

15. Immunoassay for the detection of P*l*GF in a sample, **characterized in that** a specific antibody according to one or more of Claims 2 to 13 is brought into contact with the sample and the formation of an immune complex with involvement of the P*l*GF is qualitatively or quantitatively determined.

16. Test kit for the implementation of an immunoassay according to Claim 15 containing a specific antibody according to one or more of Claims 2 to 13.

## Revendications

1. Peptide actif immunologiquement qui possède l'information de séquence d'une isoforme primaire PlGF constituée de l'une des séquences d'acide aminé suivantes :
IAN 1 : SAGNFSSEVE VVPFQEVWGR SYCRALERLV
IAN 3 : VETANVTMQL LKIRSGDRP SYVELTFSQH
ou d'une partie des peptides IAN 1 ou IAN 3, la partie contenant au moins l'une des séquences d'acide aminé VVPFQEVWGRSY (IAN : 1-1-2) ou RSGDRPSYVELT (IAN : 3-3-1).

2. Anticorps monoclonal 05-81-05, qui a été produit par la lignée cellulaire ayant la désignation 2005-81-05 enregistrée au DSMZ sous le n° d'entrée DSM ACC2764.

3. Anticorps monoclonal 05-81-10 qui a été produit par la lignée cellulaire ayant la désignation 2005-81-010 enregistrée au DSMZ sous le n° d'entrée DSM ACC2765.

4. Anticorps monoclonal 05-64/026 qui a été produit par la lignée cellulaire ayant la désignation 2005-64-026 enregistrée au DSMZ sous le n° d'entrée DSM ACC2766.

5. Anticorps monoclonal 05-54/04 qui a été produit par la lignée cellulaire ayant la désignation 2005-54/04 enregistrée au DSMZ sous le n° d'entrée DSM ACC2769.

6. Anticorps monoclonal 05-164/054 qui a été produit par la lignée cellulaire ayant la désignation 2005-164/054 enregistrée au DSMZ sous le n° d'entrée DSM ACC2770.

7. Anticorps monoclonal 05-164/042 qui a été produit par la lignée cellulaire ayant la désignation 2005-164/042 enregistrée au DSMZ sous le n° d'entrée DSM ACC2771.

8. Anticorps monoclonal 05-164/038 qui a été produit par la lignée cellulaire ayant la désignation 2005-164/038 enregistrée au DSMZ sous le n° d'entrée DSM ACC2772.

9. Anticorps monoclonal 05-164/012 qui a été produit par la lignée cellulaire ayant la désignation 2005-164/012 enregistrée au DSMZ sous le n° d'entrée DSM ACC2773.

10. Anticorps monoclonal 05-121/06 qui a été produit par la lignée cellulaire ayant la désignation 2005-121/06 enregistrée au DSMZ sous le n° d'entrée DSM ACC2774.

11. Anticorps monoclonal 05-120/03 qui a été produit par la lignée cellulaire ayant la désignation 2005-120/03 enregistrée au DSMZ sous le n° d'entrée DSM ACC2775.

12. Anticorps spécifique qui, avec l'un ou avec plusieurs des anticorps monoclonaux suivant l'une ou plusieurs des revendications 2, 3 et 6 à 11, concourt à la fixation sur du PlGF libre.

13. Anticorps spécifique qui fixe un épitope contenu dans la séquence d'acide aminé suivante : VVPFQEVWGRSY.

14. Peptide suivant la revendication 1 à utiliser comme antigène d'immunisation pour la production d'anticorps spécifiques à fPlGF.

15. Essai immunologique pour la détection de PlGF dans un échantillon **caractérisé en ce que** l'on met un anticorps spécifique suivant l'une ou plusieurs des revendications 2 à 13 en contact avec un échantillon et on détermine qualitativement ou quantitativement la formation d'un immunocomplexe avec participation du PlGF.

16. Trousse de test pour effectuer un essai immunologique suivant la revendication 15 contenant un anticorps suivant l'une ou plusieurs des revendications 2 à 13.
